# EUROPEAN PATENT APPLICATION

(11) **EP 4 596 581 A1**
(43) Date of publication of application: **06.08.2025**
(21) Application number: 23881112.9
(22) Date of filing: 04.04.2023
(51) Int. Cl.: C07K 16/28, C07K 16/46, C12N 15/13, A61K 39/395, A61P 37/02, A61P 35/00, G01N 33/68, G01N 33/574

(54) **ANTI-CD16A ANTIBODY AND APPLICATION THEREOF**

(30) Priority: 27.10.2022 CN 202211327361
(71) Applicant: Hefei TG Immunopharma Co., Ltd., Hefei, Anhui 230001 (CN)
(72) Inventor: CHENG, Ying, Hefei, Anhui 230001 (CN); CAO, Guoshuai, Hefei, Anhui 230001 (CN); WU, Yuwei, Hefei, Anhui 230001 (CN); LI, Yangyang, Hefei, Anhui 230001 (CN)
(74) Representative: Gill Jennings & Every LLP
(86) International application number: PCT/CN2023/086262
(87) International publication number: WO 2024/087515

(57) **Abstract**

Provided are an anti-CD16A antibody and an application thereof. The antibody comprises heavy chain variable regions CDR1, CDR2, CDR3 sequences as shown in SEQ ID NOs: 1, 2, and 3, or amino acid sequences at least 95% identity to SEQ ID NOs: 1, 2, and 3; and/or light chain variable regions CDR1, CDR2, CDR3 sequences as shown in SEQ ID NOs: 4, 5, and 6, respectively, or amino acid sequences at least 95% identity to SEQ ID NOs: 4, 5, and 6.

## Description

### FIELD

The present disclosure relates to the field of biomedicine, and specifically, to an anti-CD16A antibody and use thereof.

### BACKGROUND

Natural killer cells (NK cells) are important members of the innate immune system, unlike T cells, NK cells do not express antigen-specific receptors. NK cells themselves possess a broad-spectrum tumor-killing capability and play a significant role in enhancing antibody and T cell responses. Currently, NK cell-based tumor immunotherapy comes in various forms and, and the methods employed also vary. The reduction in the number of NK cells or impairment of their function is associated with the progression of various types of cancer. "Cold tumors" are immune-insensitive, and have little or no expression of MHC class I molecules on their surface, so they are hardly recognized by T cells, but they can be recognized and killed by NK cells. The introduction of such a concept has elevated the status of NK cells in antitumor immunotherapy.

The CD16 molecule is an important marker on the surface of NK cells, capable of activating the immunoreceptor tyrosine-based activation motifs (ITAMs) of IgE NK cell receptors (FcεRIγ) and CD3ζ to initiate antibody-dependent cellular cytotoxicity (ADCC). Therefore, the CD16 target is preferentially selected in bispecific antibodies based on NK cell redirection.

The CD16 target is widely selected in bispecific antibodies based on NK cell redirection (McCall et al., 1999, Gleason et al., 2014). AFM13 is a bispecific antibody targeting CD16A and CD30 molecules, which can promote the killing of CD30+ non-Hodgkin lymphoma cells by NK cells (Reusch et al., 2014, Pahl et al., 2018). In other NK cell-related bispecific antibodies, researchers utilized CD16, NKp46, and tumor targets to construct trifunctional antibodies (NKCEs). Compared with clinical therapeutic monoclonal antibodies, NKCEs exhibit a stronger in vitro killing capability and show excellent in vivo stability and tumor control (Gauthier et al., 2019).

Human CD16 can be divided into CD16A and CD 16B, of which CD16A is mainly expressed on NK cells. CD16B is mainly produced by polymorphonuclear granulocytes, and a soluble form of a receptor also presents in human serum. There are multiple alleles of CD16A in the human population. For example, CD16A has a polymorphic difference at position 158. CD16A^{158V} has a higher affinity for an Fc domain of the antibody compared with CD16A^{158F}. CD16B also has polymorphic differences in the human population, i.e., CD16B^{NA1}, CD16B^{NA2}, and CD16B^{SH}. The previous CD16 antibodies (e.g., 3G8) generally recognize CD16 rather than specifically recognizing CD16A, and thus in designing bispecific antibodies or multispecific antibodies, in order to specifically target NK cells, it is necessary to develop antibodies that recognize CD16A.

### SUMMARY

The present disclosure is based on the inventors' discovery of the following issues and facts.

NK cells themselves possess broad-spectrum tumor-killing capabilities and play an important role in enhancing antibody and T-cell responses. CD16A protein is mainly expressed in NK cells, and the CD16 target is preferentially selected in bispecific antibodies based on an NK cell redirection.

The inventors of the present disclosure successfully screened out a murine anti-CD16A monoclonal antibody that exhibits relatively high binding activity to human CD16A protein or monkey CD16A protein. Further, the inventors humanized the constant region of the above monoclonal antibody while retaining the CDR of the murine anti-CD16A monoclonal antibody to obtain a chimeric antibody. Furthermore, the framework region in a light chain variable region or a heavy chain variable region of the chimeric antibody was humanized to produce a fully humanized anti-CD16A antibody. The humanized antibody can not only specifically target and bind to human CD16A protein and monkey CD16A protein, but also has the features of low immunogenicity, and thus can effectively treat and/or prevent the CD16A-mediated disease such as an autoimmune disease.

Additionally, the bispecific binding molecules or multispecific binding molecules prepared using the anti-CD16A antibody can also specifically target and bind to human CD16A protein and monkey CD16A protein. Generally, due to the specificity of the bispecific binding molecules or multispecific binding molecules, the bispecific binding molecules or multispecific binding molecules can target the NK cells to other antigens, eliminating the cells producing such antigens through NK cell-mediated cell killing, thereby treating various diseases such as tumors.

Therefore, in a first aspect of the present disclosure, an antibody or antigen-binding fragment is provided. According to an embodiment of the present disclosure, the antibody or antigen-binding fragment includes: a heavy chain variable region CDR1 sequence, a heavy chain variable region CDR2 sequence, and a heavy chain variable region CDR3 sequence as set forth in SEQ ID NO: 1, SEQ ID NO: 2, and SEQ ID NO: 3, respectively, or as set forth in amino acid sequences having at least 95% identity to SEQ ID NO: 1, SEQ ID NO: 2, and SEQ ID NO: 3, respectively; and/or a light chain variable region CDR1 sequence, a light chain variable region CDR2 sequence, and a light chain variable region CDR3 sequence as set forth in SEQ ID NO: 4, SEQ ID NO: 5, and SEQ ID NO: 6, respectively, or as set forth in amino acid sequences having at least 95% identity to SEQ ID NO: 4, SEQ ID NO: 5, and SEQ ID NO: 6, respectively. The antibody or antigen-binding fragment according to the embodiment of the present disclosure can bind to human CD16A protein or monkey CD16A protein to effectively treat or prevent the CD16A-mediated disease.

According to an embodiment of the present disclosure, the antibody or antigen-binding fragment as described above can further include at least one of the following additional technical features.

According to an embodiment of the present disclosure, the antibody or antigen-binding fragment includes: at least one of a heavy chain framework region, FR, and a light chain FR.

According to an embodiment of the present disclosure, at least part of the at least one of the heavy chain FR and the light chain FR is derived from at least one of a human antibody, a primate antibody, a murine antibody, or a mutant thereof.

According to an embodiment of the present disclosure, the antibody or antigen-binding fragment includes: at least one of a heavy chain framework region HFR1 sequence, a heavy chain framework region HFR2 sequence, a heavy chain framework region HFR3 sequence, and a heavy chain framework region HFR4 sequence as set forth in SEQ ID NO: 7 to SEQ ID NO: 10, respectively; or at least one of a heavy chain framework region HFR1 sequence, a heavy chain framework region HFR2 sequence, a heavy chain framework region HFR3 sequence, and a heavy chain framework region HFR4 sequence as set forth in SEQ ID NO: 15 to SEQ ID NO: 18, respectively.

According to an embodiment of the present disclosure, the antibody or antigen-binding fragment includes: at least one of a light chain framework region LFR1 sequence, a light chain framework region LFR2 sequence, a light chain framework region LFR3 sequence, and a light chain framework region LFR4 sequence as set forth in SEQ ID NO: 11 to SEQ ID NO: 14, respectively; or at least one of a light chain framework region LFR1 sequence, a light chain framework region LFR2 sequence, a light chain framework region LFR3 sequence, and a light chain framework region LFR4 sequence as set forth in SEQ ID NO: 19 to SEQ ID NO: 22, respectively.

According to an embodiment of the present disclosure, the antibody or antigen-binding fragment includes: at least one of the heavy chain framework region HFR1 sequence, the heavy chain framework region HFR2 sequence, the heavy chain framework region HFR3 sequence, and the heavy chain framework region HFR4 sequence as set forth in SEQ ID NO: 7 to SEQ ID NO: 10, respectively, and at least one of the light chain framework region LFR1 sequence, the light chain framework region LFR2 sequence, the light chain framework region LFR3 sequence, and the light chain framework region LFR4 sequence as set forth in SEQ ID NO: 11 to SEQ ID NO: 14, respectively; or at least one of the heavy chain framework region HFR1 sequence, the heavy chain framework region HFR2 sequence, the heavy chain framework region HFR3 sequence, and the heavy chain framework region HFR4 sequence as set forth in SEQ ID NO: 15 to SEQ ID NO: 18, respectively, and at least one of the light chain framework region LFR1 sequence, the light chain framework region LFR2 sequence, the light chain framework region LFR3 sequence, and the light chain framework region LFR4 sequence as set forth in SEQ ID NO: 19 to SEQ ID NO: 22, respectively.

According to an embodiment of the present disclosure, the antibody or antigen-binding fragment includes: a heavy chain variable region as set forth in SEQ ID NO: 23 or SEQ ID NO: 25; and/or a light chain variable region as set forth in SEQ ID NO: 24 or SEQ ID NO: 26.

According to an embodiment of the present disclosure, the antibody or antigen-binding fragment includes:
1) the heavy chain variable region as set forth in SEQ ID NO: 23 and the light chain variable region as set forth in SEQ ID NO: 24; or
2) the heavy chain variable region as set forth in SEQ ID NO: 25 and the light chain variable region as set forth in SEQ ID NO: 26.

According to an embodiment of the present disclosure, the antibody or antigen-binding fragment includes: at least one of a heavy chain constant region and a light chain constant region. At least part of the at least one of the heavy chain constant region and the light chain constant region is derived from at least one of a human antibody, a primate antibody, a murine antibody, or a mutant thereof.

According to an embodiment of the present disclosure, the light chain constant region and the heavy chain constant region are both derived from a murine IgG antibody or a mutant thereof or a humanized IgG antibody or a mutant thereof.

According to an embodiment of the present disclosure, the light chain constant region and the heavy chain constant region are both derived from a murine IgG1 antibody or a mutant thereof or a human IgG1 antibody or a mutant thereof.

According to an embodiment of the present disclosure, the antibody includes a heavy chain constant region having an amino acid sequence as set forth in SEQ ID NO: 27 or 29 and/or a light chain constant region having an amino acid sequence as set forth in SEQ ID NO: 28 or 30.

According to an embodiment of the present disclosure, the antibody or antigen-binding fragment includes: a heavy chain having an amino acid sequence as set forth in any one of SEQ ID NO: 31, SEQ ID NO: 33, and SEQ ID NO: 35 and a light chain having an amino acid sequence as set forth in any one of SEQ ID NO: 32, SEQ ID NO: 34, and SEQ ID NO: 36.

According to an embodiment of the present disclosure, the antibody or antigen-binding fragment includes:
a heavy chain having an amino acid sequence as set forth in SEQ ID NO: 31 and a light chain of an amino acid sequence as set forth in SEQ ID NO: 32;
a heavy chain having an amino acid sequence as set forth in SEQ ID NO: 33 and a light chain of an amino acid sequence as set forth in SEQ ID NO: 34; or
a heavy chain having an amino acid sequence as set forth in SEQ ID NO: 35 and a light chain of an amino acid sequence as set forth in SEQ ID NO: 36.

According to an embodiment of the present disclosure, the antibody or antigen-binding fragment includes a monoclonal antibody or a polyclonal antibody.

According to an embodiment of the present disclosure, the monoclonal antibody includes at least one of Fv, a single-chain antibody, Fab, a single-domain antibody, and a minimal recognition unit.

According to an embodiment of the present disclosure, the antibody or antigen-binding fragment is capable of binding to an amino acid sequence as set forth in SEQ ID NO: 37 and/or 38.

In a second aspect of the present disclosure, a bispecific binding molecule is provided. According to an embodiment of the present disclosure, the bispecific binding molecule includes: a first binding region including the antibody or antigen-binding fragment as described in the first aspect; and a second binding region that have a binding activity to BCMA or B7H6. The bispecific binding molecule according to the embodiment of the present disclosure can bind to human CD16A proteins and human BCMA protein or monkey CD16A protein and monkey BCMA protein, or to human CD16A protein and human B7H6 protein or monkey CD16A protein and monkey B7H6 protein, which can be applied in scientific research, or effectively treat or prevent a CD16A and BCMA-mediated disease or a CD16A and B7H6-mediated disease.

Those skilled in the art should understand that the binding activity of the second binding region is not particularly limited. The second binding region can also have other binding activities, as long as the bispecific antibody has the antibody or antigen-binding fragment as described in the first aspect, and both the antibody or antigen-binding fragment and the second binding region can function effectively. In addition, more specific antibodies can be prepared using the antibody or antigen-binding fragment described in the present disclosure, such as a trispecific antibody, a tetraspecific antibody, and a pentaspecific antibody. Based on multi-specificity of the antibody, the antibody or antigen-binding fragment of the present disclosure can target NK cells to other antigens, and eliminate the cells producing such antigens through NK cell-mediated cell killing.

According to an embodiment of the present disclosure, the bispecific binding molecule as described above can further include at least one of the following additional technical features.

According to an embodiment of the present disclosure, the bispecific binding molecule includes a symmetric bispecific binding molecule or an asymmetric bispecific binding molecule.

According to an embodiment of the present disclosure, the bispecific binding molecule is the asymmetric bispecific binding molecule.

According to an embodiment of the present disclosure, the antibody or antigen-binding fragment is an anti-CD16A single-chain antibody.

According to an embodiment of the present disclosure, the second binding region includes at least one of a full-length antibody, Fv, a single-chain antibody, Fab, a single-domain antibody, and a minimal recognition unit that have a binding activity to BCMA or B7H6.

According to an embodiment of the present disclosure, the second binding region includes an anti-BCMA single-chain antibody or an anti-B7H6 single-chain antibody.

According to an embodiment of the present disclosure, the anti-CD16A single-chain antibody includes an anti-CD16A antibody light chain variable region and an anti-CD16A antibody heavy chain variable region, the anti-CD16A antibody heavy chain variable region having an amino acid sequence as set forth in SEQ ID NO: 23 or 25, and the anti-CD16A antibody light chain variable region having an amino acid sequence as set forth in SEQ ID NO: 24 or 26.

According to an embodiment of the present disclosure, the anti-CD16A single-chain antibody further includes a linker peptide 1. An N-terminus of the linker peptide 1 is linked to a C-terminus of the anti-CD16A antibody heavy chain variable region, and a C-terminus of the linker peptide 1 is linked to an N-terminus of the anti-CD16A antibody light chain variable region; or the N-terminus of the linker peptide 1 is linked to a C-terminus of the anti-CD16A antibody light chain variable region, and the C-terminus of the linker peptide 1 is linked to an N-terminus of the anti-CD16A antibody heavy chain variable region.

According to an embodiment of the present disclosure, the anti-BCMA single-chain antibody includes an anti-BCMA antibody light chain variable region and an anti-BCMA antibody heavy chain variable region, the anti-BCMA antibody heavy chain variable region having an amino acid sequence as set forth in SEQ ID NO: 59, and the anti-BCMA antibody light chain variable region having an amino acid sequence as set forth in SEQ ID NO: 60.

According to an embodiment of the present disclosure, the anti-BCMA single-chain antibody further includes a linker peptide 2. An N-terminus of the linker peptide 2 is linked to a C-terminus of the anti-BCMA antibody heavy chain variable region, and a C-terminus of the linker peptide 2 is linked to an N-terminus of the anti-BCMA antibody light chain variable region; or the N-terminus of the linker peptide 2 is linked to a C-terminus of the anti-BCMA antibody light chain variable region, and the C-terminus of the linker peptide 2 is linked to an N-terminus of the anti-BCMA antibody heavy chain variable region.

According to an embodiment of the present disclosure, the anti-B7H6 single-chain antibody includes an anti-B7H6 antibody light chain variable region and an anti-B7H6 antibody heavy chain variable region, the anti-B7H6 antibody heavy chain variable region having an amino acid sequence as set forth in SEQ ID NO: 61, and the anti-B7H6 antibody light chain variable region having an amino acid sequence as set forth in SEQ ID NO: 62.

According to an embodiment of the present disclosure, the anti-B7H6 single-chain antibody further includes a linker peptide 3. An N-terminus of the linker peptide 3 is linked to a C-terminus of the anti-B7H6 antibody heavy chain variable region, and a C-terminus of the linker peptide 3 is linked to an N-terminus of the anti-B7H6 antibody light chain variable region; or the N-terminus of the linker peptide 3 is linked to a C-terminus of the anti-B7H6 antibody light chain variable region, and the C-terminus of the linker peptide 3 is linked to an N-terminus of the anti-B7H6 antibody heavy chain variable region.

According to an embodiment of the present disclosure, the linker peptide 2, and the linker peptide 3 has an amino acid sequence as set forth in (GGGGS)n, where n is an integer greater than or equal to 1. Preferably, n is 1, 2, 3, 4, 5, 6, 7, 8, 9, or 10. Those skilled in the art should understand that any conventional linker peptides in the field can be used, such as a conventional flexible amino acid segment or a conventional rigid amino acid segment.

According to an embodiment of the present disclosure, at least one of the linker peptide 1, the linker peptide 2, and the linker peptide 3 has an amino acid sequence as set forth in SEQ ID NO: 44.

According to an embodiment of the present disclosure, the anti-CD16A single-chain antibody has an amino acid sequence as set forth in SEQ ID NO: 41.

According to an embodiment of the present disclosure, the anti-BCMA single-chain antibody has an amino acid sequence as set forth in SEQ ID NO: 42.

According to an embodiment of the present disclosure, the anti-B7H6 single-chain antibody has an amino acid sequence as set forth in SEQ ID NO: 43.

According to an embodiment of the present disclosure, the first binding region further includes a first Fc peptide segment, an N-terminus of the first Fc peptide segment being linked to a C-terminus of the antibody or a C-terminus of the antigen-binding fragment.

According to an embodiment of the present disclosure, the second binding region further includes a second Fc peptide segment, an N-terminus of the second Fc peptide segment being linked to a C-terminus of the anti-BCMA single-chain antibody or a C-terminus of the anti-B7H6 single-chain antibody.

According to an embodiment of the present disclosure, the first Fc peptide segment has an amino acid sequence as set forth in SEQ ID NO: 45.

According to an embodiment of the present disclosure, the second Fc peptide segment has an amino acid sequence as set forth in SEQ ID NO: 46.

According to an embodiment of the present disclosure, the first Fc peptide segment and the second Fc peptide segment are linked by a knob-into-hole structure. Those skilled in the art should understand that amino acid sequences of the first Fc peptide segment and the second Fc peptide segment may be the same or different. For example, when the amino acid sequences of the first Fc peptide segment and the second Fc peptide segment are the same, the first Fc peptide segment and the second Fc peptide segment may be linked by a disulfide bond.

According to an embodiment of the present disclosure, the first antigen-binding region has an amino acid sequence as set forth in SEQ ID NO: 47, and the second antigen-binding region has an amino acid sequence as set forth in SEQ ID NO: 48 or SEQ ID NO:49.

Nucleic acids encoding the antibody, the antigen-binding fragment, or bispecific binding molecule of the present disclosure fall within the scope of the present disclosure. Those skilled in the art can easily obtain the corresponding nucleic acid sequences based on amino acid sequences of the antibody, the antigen-binding fragment, or the bispecific binding molecule.

Therefore, in a third aspect of the present disclosure, a nucleic acid molecule is provided. The nucleic acid molecule encodes the antibody or antigen-binding fragment as described in the first aspect or the bispecific binding molecule. According to specific embodiments of the present disclosure, the antibody or antigen-binding fragment encoded by the nucleic acid molecule can bind to human CD16A protein or monkey CD16A protein to effectively treat or prevent the CD16A-mediated disease. Further, the bispecific binding molecule encoded by the nucleic acid molecule can bind to human CD16A protein and human BCMA protein or monkey CD16A protein and monkey BCMA protein, or to human CD16A protein and human B7H6 protein or monkey CD16A protein and monkey B7H6 protein to effectively treat or prevent the CD16A and BCMA-mediated disease or the CD16A and B7H6-mediated disease.

According to an embodiment of the present disclosure, the nucleic acid molecule as described above can further include at least one of the following additional technical features.

According to an embodiment of the present disclosure, the nucleic acid molecule is DNA.

It should be noted that, for the nucleic acid mentioned in the specification and the claims of the present disclosure, those skilled in the art should understand that either one or both strands of complementary double strands are actually included. For convenience, although in most cases only one strand is provided in the specification and the claims, the complementary strand is also actually disclosed. In addition, the nucleic acid sequence in the present disclosure includes both a DNA form and an RNA form, and thus the disclosure of one form implies that the other is also disclosed.

In a fourth aspect of the present disclosure, an expression vector is provided. The expression vector carries the nucleic acid molecule as described above. The expression vector may include an optional control sequence operably linked to the nucleic acid molecule. The control sequence refers to one or more control sequences that guide the expression of the nucleic acid molecule in a host. The expression vector according to the embodiment of the present disclosure can efficiently express the antibody or antigen-binding fragment in suitable host cells in large quantities.

The "Operably linked" herein means an exogenous gene is linked to a vector such that control elements in the vector, such as a transcriptional control sequence and a translational control sequence, can exert their intended function of regulating transcription and translation of the exogenous gene. When the nucleic acid molecule is linked to the vector, the nucleic acid molecule can be directly or indirectly linked to the control elements on the vector, as long as these control elements can control translation, expression, or the like of the nucleic acid molecule. Of course, these control elements may be directly derived from the vector itself or be exogenous, i.e., not derived from the vector itself. Those skilled in the art should understand that the nucleic acid molecule encoding the antibody or antigen-binding fragment can be respectively and independently inserted into different vectors, and is commonly inserted into the same vector. For example, common vectors may be plasmids, bacteriophages, etc., such as Plasmid-X.

In a fifth aspect of the present disclosure, a method for preparing the antibody or antigen-binding fragment or the bispecific binding molecule as described above is provided. The method includes: introducing the expression vector as described above into a cell; and culturing the cell under a condition suitable for protein expression and protein secretion to obtain the antibody or antigen-binding fragment or the bispecific binding molecule. According to some specific embodiments of the present disclosure, the method can efficiently obtain the antibody or antigen-binding fragment or the bispecific binding molecule in large quantities in vitro.

According to some specific embodiments of the present disclosure, the above method for preparing the antibody or antigen-binding fragment or the bispecific binding molecule can further include at least one of the following additional technical features.

According to some specific embodiments of the present disclosure, the cell is not particularly limited. Either a prokaryotic cell or a eukaryotic cell may be used.

According to some specific embodiments of the present disclosure, the cell is a eukaryotic cell.

According to some specific embodiments of the present disclosure, the eukaryotic cell is a mammalian cell. According to some specific embodiments of the present disclosure, when the cell is the eukaryotic cell, such as the mammalian cell, an expression efficiency of a recombinant antibody is relatively high.

In a sixth aspect of the present disclosure, a recombinant cell is provided. The recombinant cell carries the nucleic acid as described above, or the expression vector as described above, or is capable of expressing the antibody or antigen-binding fragment as described above or the bispecific binding molecule as described above. The recombinant cell is obtained by transfection or transformation of the expression vector. According to some specific embodiments of the present disclosure, under suitable conditions, the recombinant cell can efficiently express the antibody or antigen-binding fragment or the bispecific binding molecule in large quantities.

It should be noted that, the recombinant cell of the present disclosure is not particularly limited and may be a prokaryotic cell, a eukaryotic cell, or a bacteriophage. The prokaryotic cell may be *Escherichia coli, Bacillus subtilis, Streptomyces, Proteus mirabilis,* or like. The eukaryotic cell includes fungi such as *Pichia pastoris, Saccharomyces cerevisiae,* Schizosaccharomyces pombe, and *Trichoderma,* an insect cell such as *Spodoptera frugiperda,* a plant cell such as tobacco, and a mammalian cell such as a BHK cell, a CHO cell, a COS cell, a myeloma cell, etc. In some embodiments, the recombinant cell of the present disclosure is preferably the mammalian cell, including the BHK cell, the CHO cell, an NSO cell, or the COS cell, and does not include an animal germ cell, a fertilized egg, or an embryonic stem cell.

It should be noted that "suitable conditions" referred to in the specification of the present disclosure refer to conditions suitable for the expression of the antibody or antigen-binding fragment or the bispecific binding molecule. It is conceivable for those skilled in the art that the conditions for the expression of the antibody or antigen-binding fragment or the bispecific binding molecule include, but are not limited to, an appropriate transformation or transfection method, an appropriate transformation or transfection condition, a healthy host cell state, a suitable host cell density, a suitable cell culture environment, and a suitable cell culture duration. There is no particular limitation on "suitable conditions". Those skilled in the art can optimize the most suitable conditions for expression of the antibody or antigen-binding fragment based on specific laboratory environments.

In a seventh aspect of the present disclosure, an immunoconjugate is provided. The immunoconjugate includes the antibody or antigen-binding fragment or the bispecific binding molecule as described above and a therapeutic agent. As previously stated, according to the embodiments of the present disclosure, the antibody or antigen-binding fragment can effectively bind to the CD16A protein, and the bispecific binding molecule can bind to the human CD16A protein and the human BCMA protein or the monkey CD16A protein and the monkey BCMA protein, or to the human CD16A protein and the human B7H6 proteins or the monkey CD16A protein and the monkey B7H6 protein, effectively treating or preventing the CD16A and BCMA-mediated disease or CD16A and B7H6-mediated disease. Therefore, the immunoconjugate containing the antibody or antigen-binding fragment can also bind to human CD16A protein or monkey CD16A protein, while the immunoconjugate containing the bispecific binding molecule can also bind to the human CD16A protein and the human BCMA protein or monkey CD16A proteins and the monkey BCMA protein, or to the human CD16A protein and the human B7H6 protein or monkey CD16A protein and the monkey B7H6 protein. Such an immunoconjugate has good preventive and/or therapeutic effects on the CD16A-mediated disease, the CD16A and BCMA-mediated disease, or the CD16A and B7H6-mediated disease.

In an eighth aspect of the present disclosure, a composition is provided. The composition includes the antibody or antigen-binding fragment, the bispecific binding molecule, the nucleic acid molecule, the expression vector, or the recombinant cell as described above. As previously stated, according to some specific embodiments of the present disclosure, the antibody or antigen-binding fragment can effectively bind to the human CD16A protein or the monkey CD16A protein, and the bispecific binding molecule can bind to the human CD16A protein and the human BCMA protein or the monkey CD16A protein and the monkey BCMA protein, or to the human CD16A protein and the human B7H6 protein or the monkey CD16A protein and the monkey B7H6 protein, effectively inhibiting the proliferation of tumor cells. Therefore, the composition containing the above substances can also effectively bind to the human CD16A protein or the monkey CD16A protein, the human CD16A protein and the human BCMA protein or the monkey CD16A protein and the monkey BCMA protein, or the human CD16A protein and the human B7H6 protein or the monkey CD16A protein and the monkey B7H6 protein, and has good preventive and/or therapeutic effects on the CD16A-mediated disease, the CD16A and BCMA-mediated disease, or the CD16A and B7H6-mediated disease. A type of the composition is not particularly limited and may include a food composition or a pharmaceutical composition.

The compositions of the present disclosure may also be administered in combination with each other or with one or more other therapeutic compounds, for example, in combination with a chemotherapeutic agent. Therefore, the composition may further contain the chemotherapeutic agent. The antibody or antigen-binding fragment, or the immunoconjugate of the present disclosure may also be combined with a second therapeutic agent. Examples of the second therapeutic agent include, but are not limited to, other agents that inhibit the activity of CD16A (including other antibodies or antigen-binding fragments, peptide inhibitors, small molecule antagonists, etc.) and/or agents that interfere with upstream or downstream signal transduction of CD16A.

It should be noted that, the composition includes combinations separated in time and/or space, as long as they are capable of acting together to achieve objectives of the present disclosure. For example, components contained in the composition may be administered to a subject as a whole or separately. When the components contained in the composition are administered separately to the subject, the respective components may be administered to the subject simultaneously or sequentially.

In a ninth aspect of the present disclosure, a medicament is provided. The medicament includes the antibody or antigen-binding fragment, the bispecific binding molecule, the nucleic acid molecule, the expression vector, the recombinant cell, or the composition as described above. As previously stated, according to some specific embodiments of the present disclosure, the antibody or antigen-binding fragment can effectively bind to the human CD16A protein or monkey CD16A protein, and the bispecific binding molecule can bind to the human CD16A protein and the human BCMA protein or monkey CD16A protein and the monkey BCMA protein, or to the human CD16A protein and the human B7H6 protein or monkey CD16A protein and the monkey B7H6 protein. Therefore, the medicament containing an effective amount of active components of the antibody or antigen-binding fragment or the bispecific binding molecule, or a series of substances thereof, can also effectively bind to the human CD16A protein or monkey CD16A protein, the human CD16A protein and the human BCMA protein or monkey CD16A protein and the monkey BCMA protein, or human CD16A protein and the human B7H6 protein or monkey CD16A protein and the monkey B7H6 protein, and has good preventive and/or therapeutic effects on the CD16A-mediated disease, the CD16A and BCMA-mediated disease, or CD16A and B7H6-mediated disease.

According to an embodiment of the present disclosure, the above medicament can further include at least one of the following additional technical features.

According to an embodiment of the present disclosure, the medicament may further include a pharmaceutically acceptable carrier.

As used herein, the term "effective amount" or "effective dose" refers to an amount that produces a function or activity in humans and/or animals and is acceptable to humans and/or animals.

The effective amount of the antibody or antigen-binding fragment or the bispecific binding molecule of the present disclosure can vary with the administration mode, the severity of the disease to be treated, etc. The seletion of the preferred effective amount can be determined by those of ordinary skill in the art based on various factors (e.g., through clinical trials). Such factors include, but are not limited to: pharmacokinetic parameters of the active component such as bioavailability, metabolism, half-life period, etc.; and the severity of the disease to be treated in a patient, a weight of the patient, an immune status of the patient, a route of administration, etc. For example, depending on exigencies of the therapeutic situation, the doses can be administered several times a day separately, or the dose can be proportionally reduced.

As used herein, a "pharmaceutically acceptable" component is a substance that is suitable for use in humans and/or mammals without undue adverse side effects (e.g., toxicity, irritation, and allergic reactions), i.e., having a reasonable benefit/risk ratio. The term "pharmaceutically acceptable carrier" refers to a carrier for administration of the therapeutic agent and includes various excipients and diluents.

The medicament of the present disclosure contains a safe and effective amount of the active component of the present disclosure and the pharmaceutically acceptable carrier. Such carriers include, but not limited to, saline, buffer, glucose, water, glycerol, ethanol, and a combination thereof. Generally, a pharmaceutical preparation should be matched the administration mode, which can be oral administration, nasal administration, intradermal administration, subcutaneous administration, intramuscular administration, intravenous administration, or intraperitoneal administration. A dosage form of the medicament of the present disclosure can be an injection, an oral preparation (tablets, capsules, and oral liquids), a transdermal agent, and a sustained-release agent. For example, the medicament is prepared by conventional methods using normal saline or an aqueous solution containing glucose and other adjuvants. The medicament is preferably manufactured under sterile conditions. The antibody or antigen-binding fragment may be administered through intravenous infusion, injection, intramuscular injection, or subcutaneous injection.

Of course, the anti-CD16A monoclonal antibody or the bispecific binding molecule herein may also be formulated into part of a kit or other diagnostic reagent as desired.

In a tenth aspect of the present disclosure, a kit is provided. The kit contains the antibody or antigen-binding fragment, the bispecific binding molecule, the nucleic acid molecule, the expression vector, or the recombinant cell as described above. As previously stated, according to some specific embodiments of the present disclosure, the antibody or antigen-binding fragment can effectively bind to the human CD16A protein or the monkey CD16A protein, and the bispecific binding molecule can bind to the human CD16A protein and the human BCMA protein, or monkey CD16A protein and the monkey BCMA protein, or to the human CD16A protein and the human B7H6 protein, or monkey CD16A protein and the monkey B7H6 protein. Therefore, the kit containing the antibody or antigen-binding fragment can effectively qualitatively or quantitatively detect the human CD16A protein or the monkey CD16A protein, and the kit containing the bispecific binding molecule can effectively qualitatively or quantitatively detect the human CD16A protein and the human BCMA protein or monkey CD16A protein and the monkey BCMA protein, or human CD16A protein and the human B7H6 protein or monkey CD16A protein and the monkey B7H6 protein. The kit provided by the present disclosure can be used, for example, for immunoblotting, immunoprecipitation, and the like involving a detection using the antibody specific binding performance of human CD16A or monkey CD16A. These kits may contain any one or more of: an antagonist, an anti-CD16A antibody or a pharmaceutical reference material; a protein purification column; an immunoglobulin affinity purification buffer; an assay diluent for cells; an instruction or literature, etc. The anti-CD16A antibody can be used for different types of diagnostic tests, for example, an in vitro or in vivo detection of various diseases or a presence of a medicament, a toxin, or other proteins. For example, the anti-CD16A antibody can be used to test related diseases through detecting serum or blood of a subject, or applied in scientific research through using the kit to detect the human CD16A protein or monkey CD16A protein, the human CD16A protein and the human BCMA protein or monkey CD16A proteins and the monkey BCMA protein, or human CD16A proteins and the human B7H6 proteins or monkey CD16A proteins and the monkey B7H6 protein in a sample to be tested. Such related diseases may include CD16A-related diseases, such as autoimmune diseases or cancers. Of course, the antibody or antigen-binding fragment provided herein can also be used for radioimmunoassay and radioimmunotherapy of the above diseases. The bispecific binding molecule is also applicable to the above application scenarios and thus details thereof will be omitted herein.

The kit may further include a reagent conventionally used for detecting CD16A, or CD16A and BCMA, or CD16A and B7H6, such as a coating solution.

In an eleventh aspect of the present disclosure, a use of the antibody or antigen-binding fragment, the nucleic acid molecule, the expression vector, the recombinant cell, or the composition as described above in the preparation of a medicament is provided. The medicament is used for preventing and/or treating a CD16A-mediated disease. As previously stated, according to some specific embodiments of the present disclosure, the antibody or antigen-binding fragment can effectively bind to the human CD16A protein or monkey CD16A proteis. Therefore, the medicament containing an effective amount of the antibody or antigen-binding fragment, or a series of substances thereof, can also effectively bind to the human CD 16A protein or monkey CD16A protein, and has good preventive and/or therapeutic effects on the CD16A-mediated disease, the CD16A and BCMA-mediated disease, or the CD16A and B7H6-mediated disease.

According to an embodiment of the present disclosure, the above use in the preparation of the medicament can further include at least one of the following additional technical features.

According to an embodiment of the present disclosure, the CD16A-mediated disease includes an autoimmune disease.

The autoimmune disease includes at least one of systemic lupus erythematosus, rheumatoid arthritis, systemic vasculitis, scleroderma, dermatomyositis, autoimmune hemolytic anemia, thyroid autoimmune disease, ulcerative colitis, chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritis syndrome, pemphigus vulgaris, pemphigoid, primary biliary cirrhosis, multiple sclerosis, and acute idiopathic polyneuritis.

In a twelfth aspect of the present disclosure, use of the bispecific binding molecule, the nucleic acid molecule, the expression vector, the recombinant cell, or the composition as described above in the preparation of a medicament is provided. The medicament is used for preventing and/or treating a CD16A and BCMA-mediated disease or a CD16A and B7H6-mediated disease. As previously stated, according to some specific embodiments of the present disclosure, the bispecific binding molecule can bind to the human CD16A protein and the human BCMA protein or monkey CD16A protein and the monkey BCMA protein, or to the human CD16A protein and the human B7H6 protein or monkey CD16A protein and the monkey B7H6 protein. Therefore, the medicament containing an effective amount of active components of the bispecific binding molecule, or a series of substances thereof, can also effectively bind to the human CD16A protein and the human BCMA proteins or the monkey CD16A protein and the monkey BCMA proteins, or the human CD16A protein and the human B7H6 proteins or the monkey CD16A protein and the monkey B7H6 protein, and has good preventive and/or therapeutic effects on the CD16A and BCMA-mediated disease or the CD16A and B7H6-mediated disease.

According to an embodiment of the present disclosure, the above use in the preparation of the medicament can further include at least one of the following additional technical features.

According to an embodiment of the present disclosure, the CD16A and BCMA-mediated disease or the CD16A and B7H6-mediated disease includes a cancer.

According to an embodiment of the present disclosure, the cancer includes at least one of hemangioma, gastric cancer, liver cancer, lung cancer, breast cancer, colon cancer, nasopharyngeal cancer, bladder cancer, cervical cancer, prostate cancer, bone cancer, skin cancer, thyroid cancer, kidney cancer, esophageal cancer, melanoma, fibrosarcoma, rhabdomyosarcoma, astrocytoma, neuroblastoma, and glioma.

In a thirteenth aspect of the present disclosure, a use of the antibody or antigen-binding fragment, the nucleic acid molecule, the expression vector, or the recombinant cell as described above in the preparation of a kit is provided. The kit is used for detecting CD16A. As previously stated, according to some specific embodiments of the present disclosure, the antibody or antigen-binding fragment can effectively bind to the human CD16A proteins or the monkey CD16A proteins and block the binding of the CD16A proteins to receptors of the CD16A proteins. Therefore, the antibody or antigen-binding fragment can be used to prepare a kit for detecting the CD16A proteins. The kit can effectively qualitatively or quantitatively detect the human CD16A proteins or the monkey CD16A proteins.

In a fourteenth aspect of the present disclosure, a use of the bispecific binding molecule, the nucleic acid molecule, the expression vector, or the recombinant cell as described above in the preparation of a kit is provided. The kit is used for detecting CD16A and/or BCMA, or CD16A and/or B7H6. As previously stated, according to some specific embodiments of the present disclosure, the bispecific binding molecule can effectively bind to the human CD16A protein and the human BCMA protein or the monkey CD16A protein and the monkey BCMA protein, or to the human CD16A protein and the human B7H6 protein or the monkey CD16A protein and the monkey B7H6 protein. Therefore, the bispecific binding molecule can be used to prepare a kit for detecting CD16A and/or BCMA, or CD16A and/or B7H6. The kit can effectively qualitatively or quantitatively detect CD16A and/or BCMA, or CD16A and/or B7H6 in humans or monkeys.

In a fifteenth aspect of the present disclosure, a method for treating or preventing a CD16A-mediated disease, a CD16A and/or BCMA-mediated disease, or a CD16A and/or B7H6-mediated disease is provided. According to an embodiment of the present disclosure, the method includes administering to the subject at least one of: 1) the antibody or antigen-binding fragment as described above; 2) the bispecific binding molecule as described above; 3) the nucleic acid molecule as described above; 4) the expression vector as described above; 5) the recombinant cell as described above; 6) the composition as described above; and 7) the medicament as described above. As previously stated, the bispecific binding molecule can effectively bind to the human CD16A protein and the human BCMA protein or monkey CD16A protein and the monkey BCMA protein, or to the human CD16A protein and the human B7H6 proteins or the monkey CD16A protein and the monkey B7H6 protein, and the antibody or antigen-binding fragment can bind to the human CD16A protein or monkey CD16A protein, to effectively treat or prevent the CD16A-mediated disease, the CD16A and/or BCMA-mediated disease, or the CD16A and/or B7H6-mediated disease, preferably the autoimmune disease or the cancer. Therefore, the method according to the embodiment of the present disclosure can effectively treat or prevent the CD16A-mediated disease, the CD16A and/or BCMA-mediated disease, or the CD16A and/or B7H6-mediated disease, such as the autoimmune disease or the cancer.

According to an embodiment of the present disclosure, the above method for treating or preventing the disease can further include at least one of the following additional technical features.

According to an embodiment of the present disclosure, the CD16A-mediated disease includes an autoimmune disease.

The autoimmune disease includes at least one of systemic lupus erythematosus, rheumatoid arthritis, systemic vasculitis, scleroderma, dermatomyositis, autoimmune hemolytic anemia, thyroid autoimmune disease, ulcerative colitis, chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritis syndrome, pemphigus vulgaris, pemphigoid, primary biliary cirrhosis, multiple sclerosis, and acute idiopathic polyneuritis.

According to an embodiment of the present disclosure, the CD16A and BCMA-mediated disease or the CD16A and B7H6-mediated disease includes a cancer.

According to an embodiment of the present disclosure, the cancer includes at least one of hemangioma, gastric cancer, liver cancer, lung cancer, breast cancer, colon cancer, nasopharyngeal cancer, bladder cancer, cervical cancer, prostate cancer, bone cancer, skin cancer, thyroid cancer, kidney cancer, esophageal cancer, melanoma, fibrosarcoma, rhabdomyosarcoma, astrocytoma, neuroblastoma, and glioma.

In a sixteenth aspect of the present disclosure, a method for diagnosing a CD16A-mediated disease, a CD16A and/or BCMA-mediated disease, or a CD16A and/or B7H6-mediated disease is provided. According to an embodiment of the present disclosure, the method includes: detecting CD16A, CD16A and/or BCMA, or CD16A and/or B7H6 in a sample to be tested using at least one of: 1) the antibody or antigen-binding fragment as described above; 2) the bispecific binding molecule as described above; 3) the nucleic acid molecule as described above; 4) the expression vector as described above; and 5) the recombinant cell as described above, and determining, based on the detection result of the CD16A, the CD16A and/or the BCMA, or the CD16A and/or the B7H6, a content of the CD16A, the CD16A and/or the BCMA, or the CD16A and/or the B7H6 in the sample to be tested. In the present disclosure, the antibody or antigen-binding fragment, or the antibody or antigen-binding fragment expressed by the nucleic acid molecule, the expression vector, or the recombinant cell can effectively bind to the human CD16A protein or the monkey CD16A protein, and the bispecific binding molecule, or the bispecific binding molecule expressed by the nucleic acid molecule, the expression vector, or the recombinant cell can effectively bind to the CD16A, the CD16A and/or the BCMA, or the CD16A and/or the B7H6. Therefore, with the method of the present disclosure, the content of the CD16A, the CD16A and/or the BCMA, or the CD16A and/or the B7H6 in the sample to be tested derived from a test subject can be effectively detected, and the related disease caused by the CD16A, the CD16A and/or the BCMA, or the CD16A and/or the B7H6F can be effectively diagnosed.

According to an embodiment of the present disclosure, the above method for diagnosing the disease can further include at least one of the following additional technical features.

According to an embodiment of the present disclosure, the content of the CD16A, the CD16A and/or the BCMA, or the CD16A and/or the B7H6 in the sample to be tested being not lower than a minimum standard for the CD16A disease, the CD16A and/or the BCMA disease, or the CD16A and/or the B7H6 disease indicates that the sample to be tested is derived from a patient suffering from a related disease caused by the CD16A, the CD16A and/or the BCMA, or the CD16A and/or the B7H6. A value of the minimum standard can be determined by performing a comparative analysis and a validation on differences between the contents of the CD16A, the CD16A and/or the BCMA, or the CD16A and/or the B7H6 in the sample to be tested from a large number of individuals suffering from the related disease caused by the CD16A, the CD16A and/or the BCMA, or the CD16A and/or the B7H6 and a large number of healthy individuals.

According to an embodiment of the present disclosure, the sample to be tested includes at least one of blood, saliva, sweat, tissues, cells, serum, plasma, feces, and urine.

According to an embodiment of the present disclosure, the CD16A-mediated disease includes an autoimmune disease.

The autoimmune disease includes at least one of systemic lupus erythematosus, rheumatoid arthritis, systemic vasculitis, scleroderma, dermatomyositis, autoimmune hemolytic anemia, thyroid autoimmune disease, ulcerative colitis, chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritis syndrome, pemphigus vulgaris, pemphigoid, primary biliary cirrhosis, multiple sclerosis, and acute idiopathic polyneuritis.

According to an embodiment of the present disclosure, the CD16A and BCMA-mediated disease or the CD16A and B7H6-mediated disease includes a cancer.

According to an embodiment of the present disclosure, the cancer includes at least one of hemangioma, gastric cancer, liver cancer, lung cancer, breast cancer, colon cancer, nasopharyngeal cancer, bladder cancer, cervical cancer, prostate cancer, bone cancer, skin cancer, thyroid cancer, kidney cancer, esophageal cancer, melanoma, fibrosarcoma, rhabdomyosarcoma, astrocytoma, neuroblastoma, and glioma.

In a sixteenth aspect of the present disclosure, a method for staging a CD16A-mediated disease, a CD16A and/or BCMA-mediated disease, or a CD16A and/or B7H6F-mediated disease is provided. According to an embodiment of the present disclosure, the method includes detecting CD16A, CD16A and/or BCMA, or CD16A and/or B7H6 in a sample to be tested using at least one of: 1) the antibody or antigen-binding fragment as described above; 2) the bispecific binding molecule as described above; 3) the nucleic acid molecule as described above; 4) the expression vector as described above; and 5) the recombinant cell as described above, and determining, based on the detection result of the CD16A, the CD16A and/or the BCMA, or the CD16A and/or the B7H6, a content of the CD16A, the CD16A and/or the BCMA, or the CD16A and/or the B7H6 in the sample to be tested. In the present disclosure, the bispecific binding molecule, or the bispecific binding molecule expressed by the nucleic acid molecule, the expression vector, or the recombinant cell can effectively bind to human or monkey CD16A and/or BCMA, or human or monkey CD16A and/or B7H6, and the antibody or antigen-binding fragment, or the antibody or antigen-binding fragment expressed by the nucleic acid molecule, the expression vector, or the recombinant cell can effectively bind to the human CD16A protein or the monkey CD16A protein. Therefore, with the method of the present disclosure, the content of the CD16A, the CD16A and/or the BCMA, or the CD16A and/or the B7H6 in the sample to be tested derived from a test subject can be effectively detected, and a stage of the related disease caused by the CD16A, the CD16A and/or the BCMA, or the CD16A and/or the B7H6F can be evaluated based on the content of the CD16A, the CD16A and/or the BCMA, or the CD16A and/or the B7H6.

According to an embodiment of the present disclosure, the above method for staging the disease can further include at least one of the following additional technical features.

According to an embodiment of the present disclosure, the content of the CD16A, the CD16A and/or the BCMA, or the CD16A and/or the B7H6 in the sample to be tested being not lower than a standard level of a stage IV tumor indicates that the sample to be tested is derived from a patient suffering from the stage IV tumor; the content of the CD16A, the CD16A and/or the BCMA, or the CD16A and/or the B7H6 in the sample to be tested being between the standard levels of a stage IV tumor and a stage III tumor indicates that the sample to be tested is derived from a patient suffering from the stage III tumor; the content of the CD16A, the CD16A and/or the BCMA, or the CD16A and/or the B7H6 in the sample to be tested being between the standard levels of a stage III tumor and a stage II tumor indicates that the sample to be tested is derived from a patient suffering from the stage II tumor; and the content of the CD16A, the CD16A and/or the BCMA, or the CD16A and/or the B7H6 in the sample to be tested being between standard levels of a stage I tumor and a stage II tumor indicates that the sample to be tested is derived from a patient suffering from the stage I tumor. Those skilled in the art should understand that levels of the CD16A, the CD16A and/or the BCMA, or the CD16A and/or the B7H6 during stage I, stage II, stage III and stage IV of the tumor vary depending on tumor types. The stage of tumor can be determined by comparing the content of the CD16A, the CD16A and/or the BCMA, or the CD16A and/or the B7H6 in the sample to be tested with the standard levels of the CD16A, the CD16A and/or the BCMA, or the CD16A and/or the B7H6 at corresponding tumor stages, or with the content of the CD16A, the CD16A and/or the BCMA, or the CD16A and/or the B7H6 in samples derived from individuals or populations at the known disease stages. Values of the standard levels of stage I, stage II, stage III, and stage IV of the tumor can be determined by performing a comparative analysis and a validation on differences between the contents of the CD16A, the CD16A and/or BCMA, or CD16A and/or B7H6 in the samples to be tested from a large number of individuals suffering from an angiogenesis-related disease and a large number of healthy individuals.

According to an embodiment of the present disclosure, the sample to be tested includes at least one of blood, saliva, sweat, tissues, cells, serum, plasma, feces, and urine.

According to an embodiment of the present disclosure, the CD16A-mediated disease includes an autoimmune disease.

The autoimmune disease includes at least one of systemic lupus erythematosus, rheumatoid arthritis, systemic vasculitis, scleroderma, dermatomyositis, autoimmune hemolytic anemia, thyroid autoimmune disease, ulcerative colitis, chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritis syndrome, pemphigus vulgaris, pemphigoid, primary biliary cirrhosis, multiple sclerosis, and acute idiopathic polyneuritis.

According to an embodiment of the present disclosure, the CD16A and BCMA-mediated disease or the CD16A and B7H6-mediated disease includes a cancer.

According to an embodiment of the present disclosure, the cancer includes at least one of hemangioma, gastric cancer, liver cancer, lung cancer, breast cancer, colon cancer, nasopharyngeal cancer, bladder cancer, cervical cancer, prostate cancer, bone cancer, skin cancer, thyroid cancer, kidney cancer, esophageal cancer, melanoma, fibrosarcoma, rhabdomyosarcoma, astrocytoma, neuroblastoma, and glioma.

In a seventeenth aspect of the present disclosure, a method for assessing a prognosis of a CD16A-mediated disease, a CD16A and/or BCMA-mediated disease, or a CD16A and/or B7H6-mediated disease is provided. According to an embodiment of the present disclosure, the method includes detecting CD16A, CD16A and/or BCMA, or CD16A and/or B7H6 in a sample to be tested using at least one of: 1) the bispecific binding molecule as described above; 2) the antibody or antigen-binding fragment as described above; 3) the nucleic acid molecule as described above; 4) the expression vector as described above; and 5) the recombinant cell as described above, and determining, based on the detection result of the CD16A, the CD16A and/or the BCMA, or the CD16A and/or the B7H6, a content of the CD16A, the CD16A and/or the BCMA, or the CD16A and/or the B7H6 in the sample to be tested. As previously stated, the content of the CD16A, the CD16A and/or the BCMA, or the CD16A and/or the B7H6 has an important influence on cancer. After the individuals suffering from a related disease are treated, the prognosis of such a disease can be effectively evaluated through monitoring the content of the CD16A, the CD16A and/or the BCMA, or the CD16A and/or the B7H6 in their tissues or excretions, such as peripheral blood, urine, etc., for example, through comparing the contents of the CD16A, the CD16A and/or the BCMA, or the CD16A and/or the B7H6 in a subject before and after treatment, or comparing the content of the CD16A, the CD16A and/or the BCMA, or the CD16A and/or the B7H6 in the subject after the treatment with the content of the CD16A, the CD16A and/or the BCMA, or the CD16A and/or the B7H6 in a normal or a diseased individual. The bispecific binding molecule, or the bispecific binding molecule expressed by the nucleic acid molecule, the expression vector, or the recombinant cell can effectively bind to the CD16A and/or the BCMA, or the CD16A and/or the B7H6. The antibody or antigen-binding fragment, or the antibody or antigen-binding fragment expressed by the nucleic acid molecule, the expression vector, or the recombinant cell can effectively bind to the human CD16A or the monkey CD16A. Therefore, with the method of the present disclosure, the content of the CD16A, the CD16A and/or the BCMA, or the CD16A and/or the B7H6 in the sample to be tested derived from the subject can be effectively detected, and the prognosis of the related disease caused by the CD16A, the CD16A and/or the BCMA, or the CD16A and/or the B7H6 can be assessed based on the content of the CD16A, the CD16A and/or the BCMA, or the CD16A and/or the B7H6.

According to an embodiment of the present disclosure, the above method for assessing the prognosis of the disease can further include at least one of the following additional technical features.

According to an embodiment of the present disclosure, the sample to be tested is derived from a patient suffering from the CD16A-mediated disease, the CD16A and/or BCMA-mediated disease, or the CD16A and/or B7H6-mediated disease before or after treatment.

According to an embodiment of the present disclosure, the sample to be tested includes at least one of blood, saliva, sweat, tissues, cells, serum, plasma, feces, and urine.

According to an embodiment of the present disclosure, a prognostic effect of the CD16A-mediated disease, the CD16A and/or BCMA-mediated disease, or the CD16A and/or B7H6-mediated disease is determined based on the content of the CD16A, the CD16A and/or the BCMA, or the CD16A and/or the B7H6 in the sample to be tested of the patient suffering from the CD16A-mediated disease, the CD16A and/or BCMA-mediated disease, or the CD16A and/or B7H6-mediated disease before or after the treatment.

According to an embodiment of the present disclosure, a decrease in the content of the CD16A, the CD16A and/or the BCMA, or the CD16A and/or the B7H6 in the sample to be tested of the patient suffering from the CD16A-mediated disease, the CD16A and/or BCMA-mediated disease, or the CD16A and/or B7H6-mediated disease after treatment indicates a good prognosis for the patient.

According to an embodiment of the present disclosure, the CD16A-mediated disease includes an autoimmune disease.

The autoimmune disease includes at least one of systemic lupus erythematosus, rheumatoid arthritis, systemic vasculitis, scleroderma, dermatomyositis, autoimmune hemolytic anemia, thyroid autoimmune disease, ulcerative colitis, chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritis syndrome, pemphigus vulgaris, pemphigoid, primary biliary cirrhosis, multiple sclerosis, and acute idiopathic polyneuritis.

According to an embodiment of the present disclosure, the CD16A and BCMA-mediated disease or the CD16A and B7H6-mediated disease includes a cancer.

According to an embodiment of the present disclosure, the cancer includes at least one of hemangioma, gastric cancer, liver cancer, lung cancer, breast cancer, colon cancer, nasopharyngeal cancer, bladder cancer, cervical cancer, prostate cancer, bone cancer, skin cancer, thyroid cancer, kidney cancer, esophageal cancer, melanoma, fibrosarcoma, rhabdomyosarcoma, astrocytoma, neuroblastoma, and glioma.

In an eighteenth aspect of the present disclosure, a use of the bispecific binding molecule, the antibody or antigen-binding fragment, the nucleic acid molecule, the expression vector, the recombinant cell, the composition, or the medicament as described above in the treatment or prevention of a CD16A-mediated disease, a CD16A and/or BCMA-mediated disease, or a CD16A and/or B7H6-mediated disease is provided. As previously stated, the bispecific binding molecule has high binding activity to CD16A and/or BCMA, or CD16A and/or B7H6, and the antibody or antigen-binding fragment can effectively bind to the human CD16A or monkey CD16A, effectively treating or preventing the CD16A-mediated disease, the CD16A and/or BCMA-mediated disease, or the CD16A and/or B7H6-mediated disease.

According to an embodiment of the present disclosure, the use as described above can further include at least one of the following additional technical features.

According to an embodiment of the present disclosure, the CD16A-mediated disease includes an autoimmune disease.

The autoimmune disease includes at least one of systemic lupus erythematosus, rheumatoid arthritis, systemic vasculitis, scleroderma, dermatomyositis, autoimmune hemolytic anemia, thyroid autoimmune disease, ulcerative colitis, chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritis syndrome, pemphigus vulgaris, pemphigoid, primary biliary cirrhosis, multiple sclerosis, and acute idiopathic polyneuritis.

According to an embodiment of the present disclosure, the CD16A and BCMA-mediated disease or the CD16A and B7H6-mediated disease includes a cancer.

According to an embodiment of the present disclosure, the cancer includes at least one of hemangioma, gastric cancer, liver cancer, lung cancer, breast cancer, colon cancer, nasopharyngeal cancer, bladder cancer, cervical cancer, prostate cancer, bone cancer, skin cancer, thyroid cancer, kidney cancer, esophageal cancer, melanoma, fibrosarcoma, rhabdomyosarcoma, astrocytoma, neuroblastoma, and glioma.

In a nineteenth aspect of the present disclosure, use of the bispecific binding molecule, the antibody or antigen-binding fragment, the nucleic acid molecule, the expression vector, or the recombinant cell as described above in diagnosing, staging or assessing, or assessing a prognosis of a CD16A-mediated disease, a CD16A and/or BCMA-mediated disease, or a CD16A and/or B7H6-mediated disease is provided. As previously stated, the bispecific binding molecule, or the bispecific binding molecule expressed by the nucleic acid molecule, the expression vector, or the recombinant cell of the present disclosure can effectively bind to CD16A and/or BCMA, or CD16A and/or B7H6. The antibody or antigen-binding fragment, or the antibody or antigen-binding fragment expressed by the nucleic acid molecule, the expression vector, or the recombinant cell can effectively bind to the human CD16A or the monkey CD16A. Therefore, with the method of the present disclosure, the content of the CD16A, the CD16A and/or the BCMA, or the CD16A and/or the B7H6 in the sample to be tested derived from the subject can be effectively detected, and an effective diagnosis, staging, and a prognosis assessment can be performed on the CD16A-mediated disease, the CD16A and/or BCMA-mediated disease, or the CD16A and/or B7H6-mediated disease.

According to an embodiment of the present disclosure, the use as described above can further include at least one of the following additional technical features.

According to an embodiment of the present disclosure, the CD16A-mediated disease includes an autoimmune disease.

The autoimmune disease includes at least one of systemic lupus erythematosus, rheumatoid arthritis, systemic vasculitis, scleroderma, dermatomyositis, autoimmune hemolytic anemia, thyroid autoimmune disease, ulcerative colitis, chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritis syndrome, pemphigus vulgaris, pemphigoid, primary biliary cirrhosis, multiple sclerosis, and acute idiopathic polyneuritis.

According to an embodiment of the present disclosure, the CD16A and BCMA-mediated disease or the CD16A and B7H6-mediated disease includes a cancer.

According to an embodiment of the present disclosure, the cancer includes at least one of hemangioma, gastric cancer, liver cancer, lung cancer, breast cancer, colon cancer, nasopharyngeal cancer, bladder cancer, cervical cancer, prostate cancer, bone cancer, skin cancer, thyroid cancer, kidney cancer, esophageal cancer, melanoma, fibrosarcoma, rhabdomyosarcoma, astrocytoma, neuroblastoma, and glioma.

The term "subject" or "individual" as used in the present disclosure generally refers to a mammal, such as a primate and/or a rodent, particularly a human, a monkey, or a mouse.

The present disclosure can provide the following beneficial effects.
1) The murine anti-CD16A monoclonal antibody obtained in the present disclosure has higher CD16A protein binding activity than the existing CD16A monoclonal antibodies, and the CD16A protein includes the human CD16A protein and the monkey CD16A protein.
2) The humanized antibody obtained by humanizing the murine anti-CD16A monoclonal antibody also has higher CD16A protein binding activity than the existing CD16A monoclonal antibody, and the CD16A protein includes the human CD16A protein and the monkey CD16A protein. In addition, the humanized antibody has relatively low immunogenicity, higher safety, and longer efficacy.

Additional aspects and advantages of the present disclosure will be provided at least in part in the following description, or will become apparent at least in part from the following description, or can be learned from practicing of the present disclosure.

### BRIEF DESCRIPTION OF THE DRAWINGS

The above and/or additional aspects and advantages of the present disclosure will become apparent and easily understandable from the following description of embodiments in conjunction with the accompanying drawings, in which:
FIG. 1A is a graph showing an ELISA detection result of a murine CD16A antibody (m40F5) at different concentrations binding to a human CD16A^{158F} protein according to an embodiment of the present disclosure.
FIG. 1B is a graph showing an ELISA detection result of a murine CD16A antibody at different concentrations binding to a human CD16A^{158V} protein according to an embodiment of the present disclosure.
FIG. 1C is a graph showing an ELISA detection result of a murine CD16A antibody at different concentrations binding to a monkey CD16 protein according to an embodiment of the present disclosure.
FIG. 1D is a graph showing an ELISA detection result of a murine CD16A antibody at different concentrations binding to a human CD16B^{NA1} protein according to an embodiment of the present disclosure.
FIG. 1E is a graph showing an ELISA detection result of a murine CD16A antibody at different concentrations binding to a human CD16B^{NA2} protein according to an embodiment of the present disclosure.
FIG. 1F is a graph showing an ELISA detection result of a murine CD16A antibody at different concentrations binding to a human CD16B^{SH} protein according to an embodiment of the present disclosure.
FIG. 2A is a graph showing a detection result of a murine CD16A antibody at different concentrations binding to a CHO cell overexpressing a human CD16A protein according to an embodiment of the present disclosure.
FIG. 2B is a graph showing a detection result of a murine CD16A antibody at different concentrations binding to a CHO cell overexpressing a cynomolgus monkey CD16A protein according to an embodiment of the present disclosure.
FIG. 3A is a graph showing an ELISA detection result of a humanized CD16A antibody (h40F5) at different concentrations binding to a human CD16A^{158F} protein according to an embodiment of the present disclosure.
FIG. 3B is a graph showing an ELISA detection result of a humanized CD16A antibody (h40F5) at different concentrations binding to a human CD16A^{158V} protein according to an embodiment of the present disclosure.
FIG. 3C is a graph showing an ELISA detection result of a humanized CD16A antibody (h40F5) at different concentrations binding to a monkey CD16 protein according to an embodiment of the present disclosure.
FIG. 3D is a graph showing an ELISA detection result of a humanized CD16A antibody (h40F5) at different concentrations binding to a human CD16B^{NA1} protein according to an embodiment of the present disclosure.
FIG. 3E is a graph showing an ELISA detection result of a humanized CD16A antibody (h40F5) at different concentrations binding to a human CD16B^{NA2} protein according to an embodiment of the present disclosure.
FIG. 3F is a graph showing an ELISA detection result of a humanized CD16A antibody (h40F5) at different concentrations binding to a human CD16B^{SH} protein according to an embodiment of the present disclosure.
FIG. 4A is a graph showing a detection result of a CD16A×BCMA bispecific antibody at different concentrations binding to a human CD16A protein according to an embodiment of the present disclosure.
FIG. 4B is a graph showing a detection result of a CD16A×BCMA bispecific antibody at different concentrations binding to a human BCMA protein according to an embodiment of the present disclosure.
FIG. 5 is a graph showing a killing detection result of a CD16A×BCMA bispecific antibody at different concentrations on an NCI-H929 cell according to an embodiment of the present disclosure.
FIG. 6A is a graph showing a detection result of a CD16A×B7H6 bispecific antibody at different concentrations binding to a human CD16A protein according to an embodiment of the present disclosure.
FIG. 6B is a graph showing a detection result of a CD16A×B7H6 bispecific antibody at different concentrations binding to a human B7H6 protein according to an embodiment of the present disclosure.
FIG. 7 is a graph showing a killing detection result of a CD16A×BCMA bispecific antibody at different concentrations on an HCT-15 cell according to an embodiment of the present disclosure.

### DETAILED DESCRIPTION

Embodiments of the present disclosure are described in detail below. The embodiments described below are illustrative, and are merely used for explaining the present disclosure, and cannot be understood as a limitation to the present disclosure.

It should be noted that, terms "first" and "second" are only used for descriptive purposes, and cannot be understood as indicating or implying relative importance, or implicitly indicating the number of indicated technical features. Therefore, the features defined with "first" and "second" may explicitly or implicitly include one or more features. Further, in the description of the present disclosure, "plurality/more" means at least two, unless otherwise defined.

The endpoint values or any values of the ranges disclosed herein are not limited to the precise ranges or values. These ranges or values should be understood to include values approximating such ranges or values. For numerical ranges, one or more new numerical ranges can be obtained by combining the endpoint values of the respective ranges, an endpoint value and an individual point value within the respective ranges, and individual point values within the respective ranges with each other, and these numerical ranges should be regarded as specifically disclosed herein.

For an easier understanding of the present disclosure, certain technical and scientific terms are specifically defined below. Unless obviously and clearly defined elsewhere in this document, all other technical and scientific terms used herein have the meanings commonly understood by general technical personnel in the field to which the present disclosure belongs. Abbreviations for amino acid residues are standard 3-letter and/or 1-letter codes used in the art to refer to one of the 20 commonly used L-amino acids.

An antibody or antigen-binding fragment of the present disclosure is typically prepared by biological synthesis methods. Based on the nucleotide sequences of the present disclosure, those skilled in the art can conveniently prepare an encoding nucleic acid of the present disclosure through various known methods. These methods include, but are not limited to, PCR, DNA, etc. Specific methods can be found in Molecular Cloning: A Laboratory Manual written by J. Sambrook.

An antibody or an antigen-binding fragment of the present disclosure is typically prepared by biological synthesis methods. Based on the nucleotide sequences of the present disclosure, those skilled in the art can conveniently prepare an encoding nucleic acid of the present disclosure through various known methods. These methods include, but are not limited to, PCR, DNA artificial synthesis, etc. Specific methods can be found in Molecular Cloning: A Laboratory Manual written by J. Sambrook. As an embodiment of the present disclosure, encoding nucleic acid sequences of the present disclosure can be constructed by synthesizing nucleotide sequences by stages and performing overlap extension PCR. The antibody or antigen fragment is numbered and defined using the Kabat numbering system.

In the present disclosure, the term "monoclonal antibody" refers to an antibody having a single antigen-binding site.

In the present disclosure, the term "bispecific antibody" refers to an antibody having two different antigen-binding sites.

In the present disclosure, the term "mutant" or "variant" may refer to molecules obtained by performing mutation of one or more nucleotides or amino acids on any natural or engineered molecule.

The term "complementarity determining region" or "CDR" or "CDR sequence" refers to the amino acid sequence responsible for antigen binding in the antibody, for example, generally includes amino acid residues near 23-34 (L1), 50-56 (L2), and 89-97 (L3) in the light chain variable region, and amino acid residues near 31-35B (H1), 50-65 (H2), and 95-102 (H3) in the heavy chain variable region (Kabat et al., Sequences of Proteins of Immunological Interest, 5th Ed. Public Health Service, National Institutes of Health, Bethesda, MD.(1991)); and/or amino acid residues from the "hypervariable loops" (e.g., amino acid residues near near 26-32 (LI), 50-52 (L2), and 91-96 (L3) in the light chain variable region, and 26-32 (H1), 53-55 (H2), and 96-101 (H3) in the heavy chain variable region (Chothia and Lesk J.Mol.Biol.196: 901-917(1987)).

As used herein, when the term "identity" is used for describing amino acid sequences or nucleic acid sequences relative to reference sequences, a percentage of the same amino acid or nucleotide between two amino acid sequences or nucleic acid sequences is determined by conventional methods, for example, see Ausubel et al., eds. (1995), Current Protocols in Molecular Biology, Chapter 19 (Greene Publishing and Wiley-Interscience, New York); and the ALIGN program (Dayhoff (1978), Atlas of Protein Sequence and Structure 5: Suppl.3 (National Biomedical Research Foundation, Washington, D.C.). There are many algorithms for aligning sequences and determining sequence identity, including the homology alignment algorithm of Needleman et al., (1970) J.Mol.Biol.48:443; the local homology algorithm of Smith et al., (1981) Adv.Appl.Math.2:482; the similarity search method of Pearson et al., (1988) Proc.Natl.Acad.Sci.85:2444; the Smith-Waterman algorithm (Meth.Mol.Biol.70:173-187(1997)); and the BLASTP, BLASTN, and BLASTX algorithms (see Altschul et al., (1990) J.Mol.Biol.215:403-410). Computer programs utilizing these algorithms are also available, including but not limited to: ALIGN or Megalign (DNASTAR) software, or WU-BLAST-2 (Altschul et al., Meth.Enzym., 266:460-480(1996)); or GAP, BESTFIT, BLAST, Altschul *et al.,* ibid, FASTA, and TFASTA available in the Genetics Computing Group (GCG) package, Version 8, Madison, Wisconsin, USA; and CLUSTAL in the PC/Gene program provided by Intelligenetics, Mountain View, California.

Without substantially affecting antibody activity (remaining at least 95% of activity), substitution, addition and/or deletion of one or more amino acids (such as, 1, 2, 3, 4, 5, 6, 7, 8, 9, or 10 or more) may be conducted by those skilled in the art on the sequences in the present disclosure, to obtain variants of sequences of the antibody or functional fragments thereof.. These variants are considered to be included within the scope of the present disclosure. For example, amino acids having similar properties may be substituted in the variable region. The sequence of the variant of the present disclosure may have at least 80%, 85%, 90%, 95%, 96%, 97%, 98%, or 99% identity (or homology) to the reference sequence. Sequence identity described herein can be measured using sequence analysis software, e.g. a computer program BLAST using default parameters, in particular BLASTP or TBLASTN. Amino acid sequences referred to herein are shown in a manner from the N-terminus to the C-terminus.

As mentioned above, the monoclonal antibody of the present disclosure may be full length or may include only a functional fragment thereof (e.g., Fab, F(ab')2, or scFv fragment), or may be modified to affect the function. The present disclosure includes anti-CD16A antibodies having modified glycosylation patterns. In some applications, it may be useful to make modifications to remove undesired glycosylation sites, or the fucose moieties do not exist on the oligosaccharide chains, e.g. to enhance antibody-dependent ADCC function. In other applications, galactosylation modifications can be made to alter complement dependent cytotoxicity (CDC).

In the present disclosure, a "full-length antibody" has a tetrapeptide chain structure formed by linking two identical light chains and two identical heavy chains through interchain disulfide bonds, such as immunoglobulin G(IgG), immunoglobulin A(IgA), immunoglobulin M(IgM), immunoglobulin D(IgD), or immunoglobulin E(IgE).

The term "functional fragment" used herein refers in particular to a fragment of an antibody, such as CDR-grafted antibody, Fab, Fab', F(ab')2, Fv or scFv, nanobody, or any fragment, which can prolong the half-life through chemical modification or by incorporation into liposomes, e.g., addition of polyalkylene glycols, such as polyethylene glycol ("PEGylation, PEG") (pegylation fragment called Fv-PEG, scFv-PEG, Fab-PEG, F(ab')2-PEG or Fab'-PEG) ("PEG" is polyethylene glycol), and the fragments have CD16A binding activity. Preferably, the functional fragment will consist of or contain partial sequences of the heavy chain variable region or light chain variable region of its source antibody. The partial sequences are enough to retain the same binding specificity and sufficient affinity as its source antibody. For CD16A, it is preferred to be at least equal to 1/100 of the affinity of the source antibody, and in a more preferred manner, at least equal to 1/10. The function fragments will include at least 3 amino acids, preferably 5, 10, 15, 25, 50, and 100 contiguous amino acids of the antibody sequence from which they are derived.

In the present disclosure, unless contrary statement is given, the use term "antigen-binding fragment" generally refers to an antigen binding antibody fragment and may include a portion of an intact antibody, typically an antigen-binding region or an antigen-variable region. Exemplarily, including CDR-grafted antibody, Fab, Fab', F(ab')2, Fv or scFv, nanobody, etc.

In the present disclosure, the term "CDR-grafted antibody" refers to grafting of CDRs of a monoclonal antibody of one species into a variable region of an antibody of another species. For example, the CDRs of a murine monoclonal antibody can be grafted into the variable region of a human antibody to replace the CDRs of the humanized antibody, in such a manner that the humanized antibody acquires antigen binding specificity of the murine monoclonal antibody while reducing its heterogeneity.

As used herein, the term "Fab antibody" or "Fab" generally refers to an antibody that contains only Fab molecules, composed of VH and CH1 of a heavy chain and an intact light chain, and the light chain and the heavy chain are linked by a disulfide bond.

In the present disclosure, the term "nanobody" (single-domain antibody or VHH antibody), which was originally described as the antigen-binding immunoglobulin (variable) domain of a "heavy chain antibody" (that is, "an antibody lack of a light chain") (Hamers-Casterman C, Atarhouch T, Muyldermans S, Robinson G, Hamers C, Songa EB, Bendahman N, Hamers R.: "Naturally occurring antibodies devoid of light chains"; Nature 363, 446-448(1993)), which contains only a heavy chain variable region (VH) and conventional CH2 and CH3 regions that specifically bind to antigens via the heavy chain variable regions.

In the present disclosure, the term "Fv antibody" generally refers to an antibody consisting of only a light chain variable region (VL) and a heavy chain variable region (VH) linked by a non-covalent bond, and is the minimum functional fragment of the antibody that retains an intact antigen binding site.

In the present disclosure, the term "single chain antibody" or "scFv" refers to a fragment composed of a heavy chain variable region and a light chain variable region of an antibody linked by a short peptide.

In the present disclosure, a "knob-into-hole structure" refers to a knob-hole mutation formed in the CH3 region of the heavy chain constant region of the antibody to facilitate a heavy chain engagement and form a heterodimer, e.g., in the present disclosure, it is achieved by mutating amino acids in the CH3 domain of the human IgG1 heavy chain constant region (mutations T366S, L368A, Y407V, and Y349C in one chain, i.e., "hole", and mutations T366W and S354C in the other chain, i.e., "knob"). The numbering of the amino acids here is performed based on the Kabat numbering system. For example, "T366S" means that the amino acid T at position 366 numbered based on the Kabat numbering system is replaced by the amino acid S.

The amino acid or nucleic acid sequences involved in the present disclosure are specifically listed in Table 1.

**Table 1**

| SEQ ID NO: | Sequence | Description |
|---|---|---|
| 1 | SDYAWN | Murine m40F5 antibody heavy chain variable region |
| | | CDR1(HCDR1) |
| 2 | YISYSGYTNYNPSLES | Murine m40F5 antibody heavy chain variable region |
| | | CDR2(HCDR2) |
| 3 | EVGLRLGWFAY | Murine m40F5 antibody heavy chain variable region |
| | | CDR3(HCDR3) |
| 4 | KASQSVNNDVA | Murine m40F5 antibody light chain variable region |
| | | CDR1(LCDR1) |
| 5 | YASNRYT | Murine m40F5 antibody light chain variable region |
| | | CDR2(LCDR2) |
| 6 | QQHYSSPWT | Murine m40F5 antibody light chain variable region |
| | | CDR3(LCDR3) |
| 7 | DVQLQESGPGLVKPSQSLSLTCTVTGYSIT | Murine m40F5 antibody heavy chain variable region |
| | | FR1(mHFR1) |
| 8 | WIRQFPGNKLEWMG | Murine m40F5 antibody heavy chain variable region |
| | | FR2(mHFR2) |
| 9 | RISVTRDTSKNQFFLQLNSVTTEDTATYYCAR | Murine m40F5 antibody heavy chain variable region |
| | | FR3(mHFR3) |
| 10 | WGQGTLVAVSA | Murine m40F5 antibody heavy chain variable region |
| | | FR4(mHFR4) |
| 11 | SIVMTQTPKFLLISAGDRVTITC | Murine m40F5 antibody light chain variable region |
| | | FR1(mLFR1) |
| 12 | WYQQKPGQSPKLLIY | Murine m40F5 antibody light chain variable region |
| | | FR2(mLFR2) |
| 13 | GVPDHFTGSGYGTDFTFTISTVQAEDLAVYFC | Murine m40F5 antibody light chain variable region |
| | | FR3(mLFR3) |
| 14 | FGGGTKLEFK | Murine m40F5 antibody light chain variable region |
| | | FR4(mLFR4) |
| 15 | QVQLQESGPGLVKPSETLSLTCTVSGYSIT | Humanized h40F5 antibody heavy chain variable region |
| | | FR1(HFR1) |
| 16 | WIRQPPGKGLEWMG | Humanized h40F5 antibody heavy chain variable region |
| | | FR2(HFR2) |
| 17 | RVTISRDTSKNQFSLKLSSVTAADTAVYYCAR | Humanized h40F5 antibody heavy chain variable region |
| | | FR3(HFR3) |
| 18 | WGQGTLVTVSS | Humanized h40F5 antibody heavy chain variable region |
| | | FR4(HFR4) |
| 19 | DIQMTQSPSSLSASVGDRVTITC | Humanized h40F5 antibody light chain variable region |
| | | FR1(LFR1) |
| 20 | WYQQKPGKAPKLLIY | Humanized h40F5 antibody light chain variable region |
| | | FR2(LFR2) |
| 21 | GVPSRFSGSGSGTDFTFTISSLQPEDIATYYC | Humanized h40F5 antibody light chain variable region |
| | | FR3(LFR3) |
| 22 | FGGGTKLEIK | Humanized h40F5 antibody light chain variable region |
| | | FR4(LFR4) |
| 23 | DVQLQESGPGLVKPSQSLSLTCTVTGYSITSDYAWNWIRQFP GNKLEWMGYISYSGYTNYNPSLESRISVTRDTSKNQFFLQL NSVTTEDTATYYCAREVGLRLGWFAYWGQGTLVAVSA | Murine m40F5 antibody/human -mouse chimeric antibody ch40F5 heavy chain variable region |
| | | (m40F5-VH/ch 40F5-VH) |
| 24 | SIVMTQTPKFLLISAGDRVTITCKASQSVNNDVAWYQQKPG QSPKLLIYYASNRYTGVPDHFTGSGYGTDFTFTISTVQAEDL AVYFCQQHYSSPWTFGGGTKLEFK | Murine m40F5 antibody/human -mouse chimeric antibody ch40F5 light chain variable region |
| | | (m40F5-VL/ch 40F5-VL) |
| 25 | QVQLQESGPGLVKPSETLSLTCTVSGYSITSDYAWNWIRQPP GKGLEWMGYISYSGYTNYNPSLESRVTISRDTSKNQFSLKLS SVTAADTAVYYCAREVGLRLGWFAYWGQGTLVTVSS | Humanized h40F5 antibody heavy chain variable region |
| | | (h40F5-VH) |
| 26 | DIQMTQSPSSLSASVGDRVTITCKASQSVNNDVAWYQQKPG KAPKLLIYYASNRYTGVPSRFSGSGSGTDFTFTISSLQPEDIAT YYCQQHYSSPWTFGGGTKLEIK | Humanized h40F5 antibody light chain variable region |
| | | (h40F5-VL) |
| 27 | AKTTAPSVYPLAPVCGDTTGSSVTLGCLVKGYFPEPVTLTW NSGSLSSGVHTFPAVLQSDLYTLSSSVTVTSSTWPSQSITCNV AHPASSTKVDKKIEPRGPTIKPCPPCKCPAPNLLGGPSVFIFPP KIKDVLMISLSPIVTCVVVDVSEDDPDVQISWFVNNVEVHT AQTQTHREDYNSTLRVVSALPIQHQDWMSGKEFKCKVNNK DLPAPIERTISKPKGSVRAPQVYVLPPPEEEMTKKQVTLTCM VTDFMPEDIYVEWTNNGKTELNYKNTEPVLDSDGSYFMYS KLRVEKKNWVERNSYSCSVVHEGLHNHHTTKSFSRTPGK | Murine m40F5 antibody heavy chain constant region |
| 28 | RADAAPTVSIFPPSSEQLTSGGASVVCFLNNFYPKDINVKWK IDGSERQNGVLNSWTDQDSKDSTYSMSSTLTLTKDEYERHN SYTCEATHKTSTSPIVKSFNRNEC | Murine m40F5 antibody light chain constant region |
| 29 | | Humanized m40F5 antibody/human -mouse chimeric antibody ch40F5 heavy chain constant region |
| 30 | | Humanized m40F5 antibody/human -mouse chimeric antibody ch40F5 light chain constant region |
| 31 | DVQLQESGPGLVKPSQSLSLTCTVTGYSITSDYAWNWIRQFP GNKLEWMGYISYSGYTNYNPSLESRISVTRDTSKNQFFLQL NSVTTEDTATYYCAREVGLRLGWFAYWGQGTLVAVSAAKT TAPSVYPLAPVCGDTTGSSVTLGCLVKGYFPEPVTLTWNSG SLSSGVHTFPAVLQSDLYTLSSSVTVTSSTWPSQSITCNVAHP ASSTKVDKKIEPRGPTIKPCPPCKCPAPNLLGGPSVFIFPPKIK DVLMISLSPIVTCVVVDVSEDDPDVQISWFVNNVEVHTAQT QTHREDYNSTLRVVSALPIQHQDWMSGKEFKCKVNNKDLP APIERTISKPKGSVRAPQVYVLPPPEEEMTKKQVTLTCMVTD FMPEDIYVEWTNNGKTELNYKNTEPVLDSDGSYFMYSKLR VEKKNWVERNSYSCSVVHEGLHNHHTTKSFSRTPGK | Murine m40F5 antibody heavy chain |
| 32 | SIVMTQTPKFLLISAGDRVTITCKASQSVNNDVAWYQQKPG QSPKLLIYYASNRYTGVPDHFTGSGYGTDFTFTISTVQAEDL AVYFCQQHYSSPWTFGGGTKLEFKRADAAPTVSIFPPSSEQL TSGGASVVCFLNNFYPKDINVKWKIDGSERQNGVLNSWTD QDSKDSTYSMSSTLTLTKDEYERHNSYTCEATHKTSTSPIVK SFNRNEC | Murine m40F5 antibody light chain |
| 33 | DVQLQESGPGLVKPSQSLSLTCTVTGYSITSDYAWNWIRQFP GNKLEWMGYISYSGYTNYNPSLESRISVTRDTSKNQFFLQL NSVTTEDTATYYCAREVGLRLGWFAYWGQGTLVAVSAAST KGPSVFPLAPSSKSTSGGTAALGCLVKDYFPEPVTVSWNSG ALTSGVHTFPAVLQSSGLYSLSSVVTVPSSSLGTQTYICNVNH KPSNTKVDKKVEPKSCDKTHTCPPCPAPELLGGPSVFLFPPK PKDTLMISRTPEVTCVVVDVSHEDPEVKFNWYVDGVEVHN AKTKPREEQYNSTYRVVSVLTVLHQDWLNGKEYKCKVSNK ALPAPIEKTISKAKGQPREPQVYTLPPSRDELTKNQVSLTCLV KGFYPSDIAVEWESNGQPENNYKTTPPVLDSDGSFFLYSKLT VDKSRWQQGNVFSCSVMHEALHNHYTQKSLSLSPGK | Chimeric antibody ch40F5 heavy chain |
| 34 | SIVMTQTPKFLLISAGDRVTITCKASQSVNNDVAWYQQKPG QSPKLLIYYASNRYTGVPDHFTGSGYGTDFTFTISTVQAEDL AVYFCQQHYSSPWTFGGGTKLEFKRTVAAPSVFIFPPSDEQL KSGTASVVCLLNNFYPREAKVQWKVDNALQSGNSQESVTE QDSKDSTYSLSSTLTLSKADYEKHKVYACEVTHQGLSSPVT KSFNRGEC | Chimeric antibody ch40F5 light chain |
| 35 | QVQLQESGPGLVKPSETLSLTCTVSGYSITSDYAWNWIRQPP GKGLEWMGYISYSGYTNYNPSLESRVTISRDTSKNQFSLKLS SVTAADTAVYYCAREVGLRLGWFAYWGQGTLVTVSSASTK GPSVFPLAPSSKSTSGGTAALGCLVKDYFPEPVTVSWNSGAL TSGVHTFPAVLQSSGLYSLSSVVTVPSSSLGTQTYICNVNHK PSNTKVDKKVEPKSCDKTHTCPPCPAPELLGGPSVFLFPPKP KDTLMISRTPEVTCVVVDVSHEDPEVKFNWYVDGVEVHNA KTKPREEQYNSTYRVVSVLTVLHQDWLNGKEYKCKVSNKA LPAPIEKTISKAKGQPREPQVYTLPPSRDELTKNQVSLTCLVK GFYPSDIAVEWESNGQPENNYKTTPPVLDSDGSFFLYSKLTV DKSRWQQGNVFSCSVMHEALHNHYTQKSLSLSPGK | Humanized h40F5 antibody heavy chain |
| 36 | DIQMTQSPSSLSASVGDRVTITCKASQSVNNDVAWYQQKPG KAPKLLIYYASNRYTGVPSRFSGSGSGTDFTFTISSLQPEDIAT YYCQQHYSSPWTFGGGTKLEIKRTVAAPSVFIFPPSDEQLKS GTASVVCLLNNFYPREAKVQWKVDNALQSGNSQESVTEQD SKDSTYSLSSTLTLSKADYEKHKVYACEVTHQGLSSPVTKSF NRGEC | Humanized h40F5 antibody light chain |
| 37 | MGGGAGERLFTSSCLVGLVPLGLRISLVTCPLQCGIMWQLLL PTALLLLVSAGMRTEDLPKAVVFLEPQWYRVLEKDSVTLKC QGAYSPEDNSTQWFHNESLISSQASSYFIDAATVDDSGEYRC QTNLSTLSDPVQLEVHIGWLLLQAPRWVFKEEDPIHLRCHS WKNTALHKVTYLQNGKGRKYFHHNSDFYIPKATLKDSGSY FCRGLFGSKNVSSETVNITITQGLAVSTISSFFPPGYQVSFCLV MVLLFAVDTGLYFSVKTNIRSSTRDWKDHKFKWRKDPQDK | Human CD16A protein amino acid sequence |
| 38 | MWQLLLPTALLLLVSAGMRAEDLPKAVVFLEPQWYRVLEK DRVTLKCQGAYSPEDNSTRWFHNESLISSQTSSYFIAAARVN NSGEYRCQTSLSTLSDPVQLEVHIGWLLLQAPRWVFKEEESI HLRCHSWKNTLLHKVTYLQNGKGRKYFHQNSDFYIPKATL KDSGSYFCRGLIGSKNVSSETVNITITQDLAVSSISSFFPPGYQ VSFCLVMVLLFAVDTGLYFSMKKSIPSSTRDWEDHKFKWSK DPQDK | Monkey CD16A protein amino acid sequence |
| 39 | CAGGTGCAGCTGCAGGAGTCCGGCCCCGGCCTGGTGAAG CCCTCCGAGACCCTGTCCCTGACCTGCACCGTGTCCGGCT ACTCCATCACCTCCGACTACGCCTGGAACTGGATCAGGCA GCCCCCCGGCAAGGGCCTGGAGTGGATGGGCTACATCTC CTACTCCGGCTACACCAACTACAACCCCTCCCTGGAGTCC AGGGTGACCATCTCCAGGGACACCTCCAAGAACCAGTTC TCCCTGAAGCTGTCCTCCGTGACCGCCGCCGACACCGCC GTGTACTACTGCGCCAGGGAGGTGGGCCTGAGGCTGGGC TGGTTCGCCTACTGGGGCCAGGGCACCCTGGTGACCGTG TCCTCCGCCTCCACCAAGGGCCCCTCCGTGTTCCCCCTGG CCCCCTCCTCCAAGTCCACCTCCGGCGGCACCGCCGCCCT GGGCTGCCTGGTGAAGGACTACTTCCCCGAGCCCGTGAC CGTGTCCTGGAACTCCGGCGCCCTGACCTCCGGCGTGCA CACCTTCCCCGCCGTGCTGCAGTCCTCCGGCCTGTACTCC CTGTCCTCCGTGGTGACCGTGCCCTCCTCCTCCCTGGGCA CCCAGACCTACATCTGCAACGTGAACCACAAGCCCTCCA ACACCAAGGTGGACAAGAAGGTGGAGCCCAAGTCCTGC GACAAGACCCACACCTGCCCCCCCTGCCCCGCCCCCGAG CTGCTGGGCGGCCCCTCCGTGTTCCTGTTCCCCCCCAAGC CCAAGGACACCCTGATGATCTCCAGGACCCCCGAGGTGA CCTGCGTGGTGGTGGACGTGTCCCACGAGGACCCCGAGG TGAAGTTCAACTGGTACGTGGACGGCGTGGAGGTGCACA ACGCCAAGACCAAGCCCAGGGAGGAGCAGTACAACTCC ACCTACAGGGTGGTGTCCGTGCTGACCGTGCTGCACCAG GACTGGCTGAACGGCAAGGAGTACAAGTGCAAGGTGTCC AACAAGGCCCTGCCCGCCCCCATCGAGAAGACCATCTCC AAGGCCAAGGGCCAGCCCAGGGAGCCCCAGGTGTACACC CTGCCCCCCTCCAGGGACGAGCTGACCAAGAACCAGGTG | Nucleotide sequence encoding the humanized h40F5 antibody heavy chain |
| | TCCCTGACCTGCCTGGTGAAGGGCTTCTACCCCTCCGACA TCGCCGTGGAGTGGGAGTCCAACGGCCAGCCCGAGAACA ACTACAAGACCACCCCCCCCGTGCTGGACTCCGACGGCT CCTTCTTCCTGTACTCCAAGCTGACCGTGGACAAGTCCAG GTGGCAGCAGGGCAACGTGTTCTCCTGCTCCGTGATGCAC GAGGCCCTGCACAACCACTACACCCAGAAGTCCCTGTCC CTGTCCCCCGGCAAG | |
| 40 | GACATCCAGATGACCCAGTCCCCCTCCTCCCTGTCCGCCT CCGTGGGCGACAGGGTGACCATCACCTGCAAGGCCTCCC AGTCCGTGAACAACGACGTGGCCTGGTACCAGCAGAAGC CCGGCAAGGCCCCCAAGCTGCTGATCTACTACGCCTCCAA CAGGTACACCGGCGTGCCCTCCAGGTTCTCCGGCTCCGGC TCCGGCACCGACTTCACCTTCACCATCTCCTCCCTGCAGC CCGAGGACATCGCCACCTACTACTGCCAGCAGCACTACTC CTCCCCCTGGACCTTCGGCGGCGGCACCAAGCTGGAGAT CAAGAGGACCGTGGCCGCCCCCTCCGTGTTCATCTTCCCC CCCTCCGACGAGCAGCTGAAGTCCGGCACCGCCTCCGTG GTGTGCCTGCTGAACAACTTCTACCCCAGGGAGGCCAAG GTGCAGTGGAAGGTGGACAACGCCCTGCAGTCCGGCAAC TCCCAGGAGTCCGTGACCGAGCAGGACTCCAAGGACTCC ACCTACTCCCTGTCCTCCACCCTGACCCTGTCCAAGGCCG ACTACGAGAAGCACAAGGTGTACGCCTGCGAGGTGACCC ACCAGGGCCTGTCCTCCCCCGTGACCAAGTCCTTCAACA GGGGCGAGTGC | Nucleotide sequence encoding the humanized h40F5 antibody light chain |
| 41 | DIQMTQSPSSLSASVGDRVTITCKASQSVNNDVAWYQQKPG KAPKLLIYYASNRYTGVPSRFSGSGSGTDFTFTISSLQPEDIAT YYCQQHYSSPWTFGCGTKLEIKGGGGSGGGGSGGGGSGGG GSQVQLQESGPGLVKPSETLSLTCTVSGYSITSDYAWNWIRQ PPGKCLEWMGYISYSGYTNYNPSLESRVTISRDTSKNQFSLK LSSVTAADTAVYYCAREVGLRLGWFAYWGQGTLVTVSS | Humanized h40F5 single chain antibody |
| | | (CD16A scFv) |
| 42 | DIQMTQSPSSLSASVGDRVTITCSASQDISNYLNWYQQKPG KAPKLLIYYTSNLHSGVPSRFSGSGSGTDFTLTISSLQPEDFAT YYCQQYRKLPWTFGQGTKLEIKRGGGGSGGGGSGGGGSQ VQLVQSGAEVKKPGSSVKVSCKASGGTFSNYWMHWVRQA PGQGLEWMGATYRGHSDTYYNQKFKGRVTITADKSTSTAY MELSSLRSEDTAVYYCARGAIYDGYDVLDNWGQGTLVTVS S | BCMA single chain antibody |
| | | (BCMA scFv) |
| 43 | DIVLTQTPLSLSVTPGQPASISCKASQSVDYDGDSYMNWYL QKPGQPPQLLIYAASTLHSGIPDRFSGSGSGTDFTLKISRVEA EDVGVYYCQQSKEDPRTFGQGTKLEIKGGGGSGGGGSGGG GSEVQLVQSGAEVKKPGASVKVSCKASGYTFTDYNMDWV RQAPGQGLEWIGDINPNNGGTLYNQKFRGRVTLTVDTSISTA YMELSRLRSDDTAVYYCARSEVFYGNYADYWGQGTTVTVS S | B7H6 single chain antibody |
| | | (B7H6 scFv) |
| 44 | GGGGSGGGGSGGGGSGGGGS | Linker peptide 1 |
| 45 | EPKSCDKTHTCPPCPAPEAAGGPSVFLFPPKPKDTLMISRTPE VTCVVVDVSHEDPEVKFNWYVDGVEVHNAKTKPREEQYN STYRVVSVLTVLHQDWLNGKEYKCKVSNKALPAPIEKTISK AKGQPREPQVYTLPPCREEMTKNQVSLWCLVKGFYPSDIAV | First Fc peptide segment |
| | EWESNGQPENNYKTTPPVLDSDGSFFLYSKLTVDKSRWQQG NVFSCSVMHEALHNHYTQKSLSLSPGK | |
| 46 | EPKSCDKTHTCPPCPAPEAAGGPSVFLFPPKPKDTLMISRTPE VTCVVVDVSHEDPEVKFNWYVDGVEVHNAKTKPREEQYN STYRVVSVLTVLHQDWLNGKEYKCKVSNKALPAPIEKTISK AKGQPREPQVCTLPPSREEMTKNQVSLSCAVKGFYPSDIAVE WESNGQPENNYKTTPPVLDSDGSFFLVSKLTVDKSRWQQG NVFSCSVMHEALHNHYTQKSLSLSPGK | Second Fc peptide segment |
| 47 | DIQMTQSPSSLSASVGDRVTITCKASQSVNNDVAWYQQKPG KAPKLLIYYASNRYTGVPSRFSGSGSGTDFTFTISSLQPEDIAT YYCQQHYSSPWTFGCGTKLEIKGGGGSGGGGSGGGGSGGG GSQVQLQESGPGLVKPSETLSLTCTVSGYSITSDYAWNWIRQ PPGKCLEWMGYISYSGYTNYNPSLESRVTISRDTSKNQFSLK LSSVTAADTAVYYCAREVGLRLGWFAYWGQGTLVTVSSEP KSCDKTHTCPPCPAPEAAGGPSVFLFPPKPKDTLMISRTPEV TCVVVDVSHEDPEVKFNWYVDGVEVHNAKTKPREEQYNS TYRVVSVLTVLHQDWLNGKEYKCKVSNKALPAPIEKTISKA KGQPREPQVYTLPPCREEMTKNQVSLWCLVKGFYPSDIAVE WESNGQPENNYKTTPPVLDSDGSFFLYSKLTVDKSRWQQG NVFSCSVMHEALHNHYTQKSLSLSPGK | First antigen-binding region (CD16A scFv-Fc) |
| 48 | DIQMTQSPSSLSASVGDRVTITCSASQDISNYLNWYQQKPG KAPKLLIYYTSNLHSGVPSRFSGSGSGTDFTLTISSLQPEDFAT YYCQQYRKLPWTFGQGTKLEIKRGGGGSGGGGSGGGGSQ VQLVQSGAEVKKPGSSVKVSCKASGGTFSNYWMHWVRQA PGQGLEWMGATYRGHSDTYYNQKFKGRVTITADKSTSTAY MELSSLRSEDTAVYYCARGAIYDGYDVLDNWGQGTLVTVS SEPKSCDKTHTCPPCPAPEAAGGPSVFLFPPKPKDTLMISRTP EVTCVVVDVSHEDPEVKFNWYVDGVEVHNAKTKPREEQY NSTYRVVSVLTVLHQDWLNGKEYKCKVSNKALPAPIEKTIS KAKGQPREPQVCTLPPSREEMTKNQVSLSCAVKGFYPSDIA VEWESNGQPENNYKTTPPVLDSDGSFFLVSKLTVDKSRWQQ GNVFSCSVMHEALHNHYTQKSLSLSPGK | Second antigen-binding region (BCMA scFv-Fc) |
| 49 | DIVLTQTPLSLSVTPGQPASISCKASQSVDYDGDSYMNWYL QKPGQPPQLLIYAASTLHSGIPDRFSGSGSGTDFTLKISRVEA EDVGVYYCQQSKEDPRTFGQGTKLEIKGGGGSGGGGSGGG GSEVQLVQSGAEVKKPGASVKVSCKASGYTFTDYNMDWV RQAPGQGLEWIGDINPNNGGTLYNQKFRGRVTLTVDTSISTA YMELSRLRSDDTAVYYCARSEVFYGNYADYWGQGTTVTVS SEPKSCDKTHTCPPCPAPEAAGGPSVFLFPPKPKDTLMISRTP EVTCVVVDVSHEDPEVKFNWYVDGVEVHNAKTKPREEQY NSTYRVVSVLTVLHQDWLNGKEYKCKVSNKALPAPIEKTIS KAKGQPREPQVCTLPPSREEMTKNQVSLSCAVKGFYPSDIA VEWESNGQPENNYKTTPPVLDSDGSFFLVSKLTVDKSRWQQ GNVFSCSVMHEALHNHYTQKSLSLSPGK | Second binding region (B7H6 scFv-Fc) |
| 50 | GACGTGCAGCTGCAGGAGTCCGGCCCCGGCCTGGTGAAG CCCTCCCAGTCCCTGTCCCTGACCTGCACCGTGACCGGCT ACTCCATCACCTCCGACTACGCCTGGAACTGGATCAGGCA GTTCCCCGGCAACAAGCTGGAGTGGATGGGCTACATCTCC TACTCCGGCTACACCAACTACAACCCCTCCCTGGAGTCCA | Nucleotide sequence encoding the murine m40F5 antibody heavy |
| | GGATCTCCGTGACCAGGGACACCTCCAAGAACCAGTTCT TCCTGCAGCTGAACTCCGTGACCACCGAGGACACCGCCA CCTACTACTGCGCCAGGGAGGTGGGCCTGAGGCTGGGCT GGTTCGCCTACTGGGGCCAGGGCACCCTGGTGGCCGTGT CCGCCGCCAAGACCACCGCCCCCTCCGTGTACCCCCTGGC CCCCGTGTGCGGCGACACCACCGGCTCCTCCGTGACCCT GGGCTGCCTGGTGAAGGGCTACTTCCCCGAGCCCGTGAC CCTGACCTGGAACTCCGGCTCCCTGTCCTCCGGCGTGCAC ACCTTCCCCGCCGTGCTGCAGTCCGACCTGTACACCCTGT CCTCCTCCGTGACCGTGACCTCCTCCACCTGGCCCTCCCA GTCCATCACCTGCAACGTGGCCCACCCCGCCTCCTCCACC AAGGTGGACAAGAAGATCGAGCCCAGGGGCCCCACCATC AAGCCCTGCCCCCCCTGCAAGTGCCCCGCCCCCAACCTG CTGGGCGGCCCCTCCGTGTTCATCTTCCCCCCCAAGATCA AGGACGTGCTGATGATCTCCCTGTCCCCCATCGTGACCTG CGTGGTGGTGGACGTGTCCGAGGACGACCCCGACGTGCA GATCTCCTGGTTCGTGAACAACGTGGAGGTGCACACCGC CCAGACCCAGACCCACAGGGAGGACTACAACTCCACCCT GAGGGTGGTGTCCGCCCTGCCCATCCAGCACCAGGACTG GATGTCCGGCAAGGAGTTCAAGTGCAAGGTGAACAACAA GGACCTGCCCGCCCCCATCGAGAGGACCATCTCCAAGCC CAAGGGCTCCGTGAGGGCCCCCCAGGTGTACGTGCTGCC CCCCCCCGAGGAGGAGATGACCAAGAAGCAGGTGACCCT GACCTGCATGGTGACCGACTTCATGCCCGAGGACATCTAC GTGGAGTGGACCAACAACGGCAAGACCGAGCTGAACTAC AAGAACACCGAGCCCGTGCTGGACTCCGACGGCTCCTAC TTCATGTACTCCAAGCTGAGGGTGGAGAAGAAGAACTGG GTGGAGAGGAACTCCTACTCCTGCTCCGTGGTGCACGAG GGCCTGCACAACCACCACACCACCAAGTCCTTCTCCAGG ACCCCCGGCAAG | chain |
| 51 | TCCATCGTGATGACCCAGACCCCCAAGTTCCTGCTGATCT CCGCCGGCGACAGGGTGACCATCACCTGCAAGGCCTCCC AGTCCGTGAACAACGACGTGGCCTGGTACCAGCAGAAGC CCGGCCAGTCCCCCAAGCTGCTGATCTACTACGCCTCCAA CAGGTACACCGGCGTGCCCGACCACTTCACCGGCTCCGG CTACGGCACCGACTTCACCTTCACCATCTCCACCGTGCAG GCCGAGGACCTGGCCGTGTACTTCTGCCAGCAGCACTACT CCTCCCCCTGGACCTTCGGCGGCGGCACCAAGCTGGAGT TCAAGAGGGCCGACGCCGCCCCCACCGTGTCCATCTTCCC CCCCTCCTCCGAGCAGCTGACCTCCGGCGGCGCCTCCGTG GTGTGCTTCCTGAACAACTTCTACCCCAAGGACATCAACG TGAAGTGGAAGATCGACGGCTCCGAGAGGCAGAACGGC GTGCTGAACTCCTGGACCGACCAGGACTCCAAGGACTCC ACCTACTCCATGTCCTCCACCCTGACCCTGACCAAGGACG AGTACGAGAGGCACAACTCCTACACCTGCGAGGCCACCC ACAAGACCTCCACCTCCCCCATCGTGAAGTCCTTCAACAG GAACGAGTGC | Nucleotide sequence encoding the murine m40F5 antibody light chain |
| 52 | GACGTGCAGCTGCAGGAGTCCGGCCCCGGCCTGGTGAAG CCCTCCCAGTCCCTGTCCCTGACCTGCACCGTGACCGGCT ACTCCATCACCTCCGACTACGCCTGGAACTGGATCAGGCA | Nucleotide sequence encoding the |
| | GTTCCCCGGCAACAAGCTGGAGTGGATGGGCTACATCTCC TACTCCGGCTACACCAACTACAACCCCTCCCTGGAGTCCA GGATCTCCGTGACCAGGGACACCTCCAAGAACCAGTTCT TCCTGCAGCTGAACTCCGTGACCACCGAGGACACCGCCA CCTACTACTGCGCCAGGGAGGTGGGCCTGAGGCTGGGCT GGTTCGCCTACTGGGGCCAGGGCACCCTGGTGGCCGTGT CCGCCGCCTCCACCAAGGGCCCCTCCGTGTTCCCCCTGGC CCCCTCCTCCAAGTCCACCTCCGGCGGCACCGCCGCCCTG GGCTGCCTGGTGAAGGACTACTTCCCCGAGCCCGTGACC GTGTCCTGGAACTCCGGCGCCCTGACCTCCGGCGTGCAC ACCTTCCCCGCCGTGCTGCAGTCCTCCGGCCTGTACTCCC TGTCCTCCGTGGTGACCGTGCCCTCCTCCTCCCTGGGCAC CCAGACCTACATCTGCAACGTGAACCACAAGCCCTCCAA CACCAAGGTGGACAAGAAGGTGGAGCCCAAGTCCTGCG ACAAGACCCACACCTGCCCCCCCTGCCCCGCCCCCGAGC TGCTGGGCGGCCCCTCCGTGTTCCTGTTCCCCCCCAAGCC CAAGGACACCCTGATGATCTCCAGGACCCCCGAGGTGAC CTGCGTGGTGGTGGACGTGTCCCACGAGGACCCCGAGGT GAAGTTCAACTGGTACGTGGACGGCGTGGAGGTGCACAA CGCCAAGACCAAGCCCAGGGAGGAGCAGTACAACTCCAC CTACAGGGTGGTGTCCGTGCTGACCGTGCTGCACCAGGA CTGGCTGAACGGCAAGGAGTACAAGTGCAAGGTGTCCAA CAAGGCCCTGCCCGCCCCCATCGAGAAGACCATCTCCAA GGCCAAGGGCCAGCCCAGGGAGCCCCAGGTGTACACCCT GCCCCCCTCCAGGGACGAGCTGACCAAGAACCAGGTGTC CCTGACCTGCCTGGTGAAGGGCTTCTACCCCTCCGACATC GCCGTGGAGTGGGAGTCCAACGGCCAGCCCGAGAACAA CTACAAGACCACCCCCCCCGTGCTGGACTCCGACGGCTC CTTCTTCCTGTACTCCAAGCTGACCGTGGACAAGTCCAGG TGGCAGCAGGGCAACGTGTTCTCCTGCTCCGTGATGCACG AGGCCCTGCACAACCACTACACCCAGAAGTCCCTGTCCC TGTCCCCCGGCAAG | human-mouse chimeric antibody ch40F5 antibody heavy chain |
| 53 | TCCATCGTGATGACCCAGACCCCCAAGTTCCTGCTGATCT CCGCCGGCGACAGGGTGACCATCACCTGCAAGGCCTCCC AGTCCGTGAACAACGACGTGGCCTGGTACCAGCAGAAGC CCGGCCAGTCCCCCAAGCTGCTGATCTACTACGCCTCCAA CAGGTACACCGGCGTGCCCGACCACTTCACCGGCTCCGG CTACGGCACCGACTTCACCTTCACCATCTCCACCGTGCAG GCCGAGGACCTGGCCGTGTACTTCTGCCAGCAGCACTACT CCTCCCCCTGGACCTTCGGCGGCGGCACCAAGCTGGAGT TCAAGAGGACCGTGGCCGCCCCCTCCGTGTTCATCTTCCC CCCCTCCGACGAGCAGCTGAAGTCCGGCACCGCCTCCGT GGTGTGCCTGCTGAACAACTTCTACCCCAGGGAGGCCAA GGTGCAGTGGAAGGTGGACAACGCCCTGCAGTCCGGCAA CTCCCAGGAGTCCGTGACCGAGCAGGACTCCAAGGACTC CACCTACTCCCTGTCCTCCACCCTGACCCTGTCCAAGGCC GACTACGAGAAGCACAAGGTGTACGCCTGCGAGGTGACC CACCAGGGCCTGTCCTCCCCCGTGACCAAGTCCTTCAACA GGGGCGAGTGC | Nucleotide sequence encoding the human-mouse chimeric antibody ch40F5 antibody light chain |
| 54 | GACATCCAGATGACCCAGTCCCCCTCCTCCCTGTCCGCCT | Nucleotide |
| | CCGTGGGCGACAGGGTGACCATCACCTGCAAGGCCTCCC AGTCCGTGAACAACGACGTGGCCTGGTACCAGCAGAAGC CCGGCAAGGCCCCCAAGCTGCTGATCTACTACGCCTCCAA CAGGTACACCGGCGTGCCCTCCAGGTTCTCCGGCTCCGGC TCCGGCACCGACTTCACCTTCACCATCTCCTCCCTGCAGC CCGAGGACATCGCCACCTACTACTGCCAGCAGCACTACTC CTCCCCCTGGACCTTCGGCTGCGGCACCAAGCTGGAGATC AAGGGCGGCGGCGGCTCCGGCGGCGGCGGCTCCGGCGG CGGCGGCTCCGGCGGCGGCGGCTCCCAGGTGCAGCTGCA GGAGTCCGGCCCCGGCCTGGTGAAGCCCTCCGAGACCCT GTCCCTGACCTGCACCGTGTCCGGCTACTCCATCACCTCC GACTACGCCTGGAACTGGATCAGGCAGCCCCCCGGCAAG TGCCTGGAGTGGATGGGCTACATCTCCTACTCCGGCTACA CCAACTACAACCCCTCCCTGGAGTCCAGGGTGACCATCTC CAGGGACACCTCCAAGAACCAGTTCTCCCTGAAGCTGTC CTCCGTGACCGCCGCCGACACCGCCGTGTACTACTGCGCC AGGGAGGTGGGCCTGAGGCTGGGCTGGTTCGCCTACTGG GGCCAGGGCACCCTGGTGACCGTGTCCTCCGAGCCCAAG TCCTGCGACAAGACCCACACCTGCCCCCCCTGCCCCGCC CCCGAGGCCGCCGGCGGCCCCTCCGTGTTCCTGTTCCCCC CCAAGCCCAAGGACACCCTGATGATCTCCAGGACCCCCG AGGTGACCTGCGTGGTGGTGGACGTGTCCCACGAGGACC CCGAGGTGAAGTTCAACTGGTACGTGGACGGCGTGGAGG TGCACAACGCCAAGACCAAGCCCAGGGAGGAGCAGTAC AACTCCACCTACAGGGTGGTGTCCGTGCTGACCGTGCTGC ACCAGGACTGGCTGAACGGCAAGGAGTACAAGTGCAAG GTGTCCAACAAGGCCCTGCCCGCCCCCATCGAGAAGACC ATCTCCAAGGCCAAGGGCCAGCCCAGGGAGCCCCAGGTG TACACCCTGCCCCCCTGCAGGGAGGAGATGACCAAGAAC CAGGTGTCCCTGTGGTGCCTGGTGAAGGGCTTCTACCCCT CCGACATCGCCGTGGAGTGGGAGTCCAACGGCCAGCCCG AGAACAACTACAAGACCACCCCCCCCGTGCTGGACTCCG ACGGCTCCTTCTTCCTGTACTCCAAGCTGACCGTGGACAA GTCCAGGTGGCAGCAGGGCAACGTGTTCTCCTGCTCCGT GATGCACGAGGCCCTGCACAACCACTACACCCAGAAGTC CCTGTCCCTGTCCCCCGGCAAG | sequence encoding the first binding region (CD16A scFv-Fc) |
| 55 | GACATCCAGATGACCCAGTCCCCCTCCCTGTCCGCCT CCGTGGGCGACAGGGTGACCATCACCTGCTCCGCCTCCC AGGACATCTCCAACTACCTGAACTGGTACCAGCAGAAGC CCGGCAAGGCCCCCAAGCTGCTGATCTACTACACCTCCAA CCTGCACTCCGGCGTGCCCTCCAGGTTCTCCGGCTCCGGC TCCGGCACCGACTTCACCCTGACCATCTCCTCCCTGCAGC CCGAGGACTTCGCCACCTACTACTGCCAGCAGTACAGGA AGCTGCCCTGGACCTTCGGCCAGGGCACCAAGCTGGAGA TCAAGAGGGGCGGCGGCGGCTCCGGCGGCGGCGGCTCCG GCGGCGGCGGCTCCCAGGTGCAGCTGGTGCAGTCCGGCG CCGAGGTGAAGAAGCCCGGCTCCTCCGTGAAGGTGTCCT GCAAGGCCTCCGGCGGCACCTTCTCCAACTACTGGATGCA CTGGGTGAGGCAGGCCCCCGGCCAGGGCCTGGAGTGGAT GGGCGCCACCTACAGGGGCCACTCCGACACCTACTACAA | Nucleotide sequence encoding the second binding region (BCMA scFv-Fc) |
| | CCAGAAGTTCAAGGGCAGGGTGACCATCACCGCCGACAA GTCCACCTCCACCGCCTACATGGAGCTGTCCTCCCTGAGG TCCGAGGACACCGCCGTGTACTACTGCGCCAGGGGCGCC ATCTACGACGGCTACGACGTGCTGGACAACTGGGGCCAG GGCACCCTGGTGACCGTGTCCTCCGAGCCCAAGTCCTGC GACAAGACCCACACCTGCCCCCCCTGCCCCGCCCCCGAG GCCGCCGGCGGCCCCTCCGTGTTCCTGTTCCCCCCCAAGC CCAAGGACACCCTGATGATCTCCAGGACCCCCGAGGTGA CCTGCGTGGTGGTGGACGTGTCCCACGAGGACCCCGAGG TGAAGTTCAACTGGTACGTGGACGGCGTGGAGGTGCACA ACGCCAAGACCAAGCCCAGGGAGGAGCAGTACAACTCC ACCTACAGGGTGGTGTCCGTGCTGACCGTGCTGCACCAG GACTGGCTGAACGGCAAGGAGTACAAGTGCAAGGTGTCC AACAAGGCCCTGCCCGCCCCCATCGAGAAGACCATCTCC AAGGCCAAGGGCCAGCCCAGGGAGCCCCAGGTGTGCAC CCTGCCCCCCTCCAGGGAGGAGATGACCAAGAACCAGGT GTCCCTGTCCTGCGCCGTGAAGGGCTTCTACCCCTCCGAC ATCGCCGTGGAGTGGGAGTCCAACGGCCAGCCCGAGAAC AACTACAAGACCACCCCCCCCGTGCTGGACTCCGACGGC TCCTTCTTCCTGGTGTCCAAGCTGACCGTGGACAAGTCCA GGTGGCAGCAGGGCAACGTGTTCTCCTGCTCCGTGATGC ACGAGGCCCTGCACAACCACTACACCCAGAAGTCCCTGT CCCTGTCCCCCGGCAAG | |
| 56 | GACATCGTGCTGACCCAGACCCCCCTGTCCCTGTCCGTGA CCCCCGGCCAGCCCGCCTCCATCTCCTGCAAGGCCTCCCA GTCCGTGGACTACGACGGCGACTCCTACATGAACTGGTAC CTGCAGAAGCCCGGCCAGCCCCCCCAGCTGCTGATCTAC GCCGCCTCCACCCTGCACTCCGGCATCCCCGACAGGTTCT CCGGCTCCGGCTCCGGCACCGACTTCACCCTGAAGATCTC CAGGGTGGAGGCCGAGGACGTGGGCGTGTACTACTGCCA GCAGTCCAAGGAGGACCCCAGGACCTTCGGCCAGGGCAC CAAGCTGGAGATCAAGGGCGGCGGCGGCTCCGGCGGCGG CGGCTCCGGCGGCGGCGGCTCCGAGGTGCAGCTGGTGCA GTCCGGCGCCGAGGTGAAGAAGCCCGGCGCCTCCGTGAA GGTGTCCTGCAAGGCCTCCGGCTACACCTTCACCGACTAC AACATGGACTGGGTGAGGCAGGCCCCCGGCCAGGGCCTG GAGTGGATCGGCGACATCAACCCCAACAACGGCGGCACC CTGTACAACCAGAAGTTCAGGGGCAGGGTGACCCTGACC GTGGACACCTCCATCTCCACCGCCTACATGGAGCTGTCCA GGCTGAGGTCCGACGACACCGCCGTGTACTACTGCGCCA GGTCCGAGGTGTTCTACGGCAACTACGCCGACTACTGGG GCCAGGGCACCACCGTGACCGTGTCCTCCGAGCCCAAGT CCTGCGACAAGACCCACACCTGCCCCCCCTGCCCCGCCC CCGAGGCCGCCGGCGGCCCCTCCGTGTTCCTGTTCCCCCC CAAGCCCAAGGACACCCTGATGATCTCCAGGACCCCCGA GGTGACCTGCGTGGTGGTGGACGTGTCCCACGAGGACCC CGAGGTGAAGTTCAACTGGTACGTGGACGGCGTGGAGGT GCACAACGCCAAGACCAAGCCCAGGGAGGAGCAGTACA ACTCCACCTACAGGGTGGTGTCCGTGCTGACCGTGCTGCA CCAGGACTGGCTGAACGGCAAGGAGTACAAGTGCAAGGT | Nucleotide sequence encoding the second binding region (B7H6 scFv-Fc) |
| | GTCCAACAAGGCCCTGCCCGCCCCCATCGAGAAGACCAT CTCCAAGGCCAAGGGCCAGCCCAGGGAGCCCCAGGTGTG CACCCTGCCCCCCTCCAGGGAGGAGATGACCAAGAACCA GGTGTCCCTGTCCTGCGCCGTGAAGGGCTTCTACCCCTCC GACATCGCCGTGGAGTGGGAGTCCAACGGCCAGCCCGAG AACAACTACAAGACCACCCCCCCCGTGCTGGACTCCGAC GGCTCCTTCTTCCTGGTGTCCAAGCTGACCGTGGACAAGT CCAGGTGGCAGCAGGGCAACGTGTTCTCCTGCTCCGTGAT GCACGAGGCCCTGCACAACCACTACACCCAGAAGTCCCT GTCCCTGTCCCCCGGCAAG | |
| 57 | ATGGGTGGAGGGGCTGGGGAAAGGCTGTTTACTTCCTCCT GTCTAGTCGGTTTGGTCCCTTTAGGGCTCCGGATATCTTTG GTGACTTGTCCACTCCAGTGTGGCATCATGTGGCAGCTGC TCCTCCCAACTGCTCTGCTACTTCTAGTTTCAGCTGGCATG CGGACTGAAGATCTCCCAAAGGCTGTGGTGTTCCTGGAG CCTCAATGGTACAGGGTGCTCGAGAAGGACAGTGTGACT CTGAAGTGCCAGGGAGCCTACTCCCCTGAGGACAATTCC ACACAGTGGTTTCACAATGAGAGCCTCATCTCAAGCCAG GCCTCGAGCTACTTCATTGACGCTGCCACAGTCGACGACA GTGGAGAGTACAGGTGCCAGACAAACCTCTCCACCCTCA GTGACCCGGTGCAGCTAGAAGTCCATATCGGCTGGCTGTT GCTCCAGGCCCCTCGGTGGGTGTTCAAGGAGGAAGACCC TATTCACCTGAGGTGTCACAGCTGGAAGAACACTGCTCTG CATAAGGTCACATATTTACAGAATGGCAAAGGCAGGAAGT ATTTTCATCATAATTCTGACTTCTACATTCCAAAAGCCACA CTCAAAGACAGCGGCTCCTACTTCTGCAGGGGGCTTTTTG GGAGTAAAAATGTGTCTTCAGAGACTGTGAACATCACCAT CACTCAAGGTTTGGCAGTGTCAACCATCTCATCATTCTTTC CACCTGGGTACCAAGTCTCTTTCTGCTTGGTGATGGTACT CCTTTTTGCAGTGGACACAGGACTATATTTCTCTGTGAAG ACAAACATTCGAAGCTCAACAAGAGACTGGAAGGACCAT AAATTTAAATGGAGAAAGGACCCTCAAGACAAA | Nucleotide sequence encoding the human CD16A protein |
| 58 | ATGTGGCAGCTGCTCCTCCCAACTGCTCTGCTACTTCTAG TTTCAGCTGGCATGCGGGCTGAAGATCTCCCAAAGGCTGT GGTGTTCCTGGAGCCTCAATGGTACAGGGTGCTCGAGAA GGACCGTGTGACTCTGAAGTGCCAGGGAGCCTACTCCCC TGAGGACAATTCCACACGGTGGTTTCACAATGAGAGCCTC ATCTCAAGCCAGACCTCGAGCTACTTCATTGCTGCTGCCA GAGTCAACAACAGTGGAGAGTACAGGTGCCAGACAAGC CTCTCCACACTCAGTGACCCGGTGCAGCTGGAAGTCCATA TCGGCTGGCTATTGCTCCAGGCCCCTCGGTGGGTGTTCAA GGAGGAAGAATCTATTCACCTGAGGTGTCACAGCTGGAA GAACACTCTTCTGCATAAGGTCACGTATTTACAGAATGGC AAAGGCAGGAAGTATTTTCATCAGAATTCTGACTTCTACA TTCCAAAAGCCACACTCAAAGACAGCGGCTCCTACTTCT GCAGGGGACTTATTGGGAGTAAAAATGTATCTTCAGAGAC TGTGAACATCACCATCACTCAAGATTTGGCAGTGTCATCC ATCTCATCATTCTTTCCACCTGGGTACCAAGTCTCTTTCTG CCTGGTGATGGTACTCCTTTTTGCAGTGGACACAGGACTA TATTTCTCTATGAAGAAAAGCATTCCAAGCTCAACAAGGG | Nucleotide sequence encoding cynomolgus monkey CD16 protein |
| | ACTGGGAGGACCATAAATTTAAATGGAGCAAGGACCCTC AAGACAAATGA | |
| 59 | QVQLVQSGAEVKKPGSSVKVSCKASGGTFSNYWMHWVRQ APGQGLEWMGATYRGHSDTYYNQKFKGRVTITADKSTSTA YMELSSLRSEDTAVYYCARGAIYDGYDVLDNWGQGTLVTV SS | Anti-BCMA |
| | | antibody heavy chain variable region |
| 60 | DIVLTQTPLSLSVTPGQPASISCKASQSVDYDGDSYMNWYL QKPGQPPQLLIYAASTLHSGIPDRFSGSGSGTDFTLKISRVEA EDVGVYYCQQSKEDPRTFGQGTKLEIK | Anti-BCMA |
| | | antibody light chain variable region |
| 61 | EVQLVQSGAEVKKPGASVKVSCKASGYTFTDYNMDWVRQ APGQGLEWIGDINPNNGGTLYNQKFRGRVTLTVDTSISTAY MELSRLRSDDTAVYYCARSEVFYGNYADYWGQGTTVTVSS | Anti-B7H6 |
| | | antibody heavy chain variable region |
| 62 | DIVLTQTPLSLSVTPGQPASISCKASQSVDYDGDSYMNWYL QKPGQPPQLLIYAASTLHSGIPDRFSGSGSGTDFTLKISRVEA EDVGVYYCQQSKEDPRTFGQGTKLEIK | Anti-B7H6 |
| | | antibody light chain variable region |

Hereinafter, the present disclosure will be described in detail by way of examples. In the examples or test examples, the experimental methods without specifying the specific conditions were carried out according to conventional conditions.

Hereinafter, the scheme of the present disclosure will be explained in conjunction with examples. Those skilled in the art will understand that the following examples are only used to illustrate the present disclosure and should not be considered as limiting the scope of the present disclosure. Where specific techniques or conditions are not specified in the examples, they are performed according to techniques or conditions described in the literature in the art or according to the product specification. The reagents or instruments used without specifying the manufacturer are all conventional products that can be obtained commercially.

### Example 1: Construction of CHO-K1 cells overexpressing human and cynomolgus monkey CD16A

HEK293T cells were plated in a T150 flask and cultured with a DMEM complete medium. After overnight culture, endotoxin-free psPAX2, pMD2.G, and pCDH-CMV-FCGR3A-IRES-puro vector (the coding sequence (SEQ ID NO: 57) of the human CD16A protein (SEQ ID NO: 37 or the coding sequence (SEQ ID NO: 58) of the cynomolgus monkey protein (SEQ ID NO: 38) was inserted between multiple cloning sites of the vector pCDH-CMV-MCS-IRES-puro) were added to 1.5 mL Opti-MEM (Gibco, Catalog No. 31985070) medium at a ratio of 7:3:10. Then, 100 µL of P3000 transfection reagent was added. 100 µL of Lipofectamine 3000 (thermo, Catalog No. L300008) transfection reagent was taken and added to 1.5 mL Opti-MEM medium and mixed well. The DNA dilution and the liposome dilution were mixed evenly at a volume ratio of 1:1, incubated at a room temperature for 5 min to 10 min, and then added to 293T cells. The virus supernatant was harvested after 48 hours of culture. The virus supernatant was centrifuged at 2,000 g for 10 min at 4°C. The supernatant was taken and filtered through a 0.45 µm filter, and then PEG8000 solution (Sangon Biotech) was added and mixed thoroughly. The mixture was placed at 4°C overnight, and then centrifuged at 2,200 g for 90 min. A white precipitate was formed at a bottom of the centrifuge tube. The virus was resuspended in sterile PBS buffer.

Polybrene (sigma, TR-1003) (a final concentration of 8 µg/mL) was added to DME/F12 medium and mixed well. Then, an appropriate amount of virus solution was added. 2E5 CHO-K1 cells were taken and placed in a 24-well plate. A virus-containing medium was added. The plate was placed in an incubator for 8 hours, then the medium was replaced with a fresh medium. After 48 hours, an expression level of CD16A on a surface of the CHO-K1 cell was detected by flow cytometry assay. After positive cell populations appeared, limited dilution was carried out, that is, the cells were digested and diluted to a density of 4 cells per milliliter, and then inoculated into a 96-well flat-bottom plate, with 200 µl added to each well. When the cells clearly formed single cell clusters after 2 weeks of culture, the expression level of CD16A on the surface of each cloned cell was detected by the flow cytometry assay. The fully positive cells were CHO-K1 cells overexpressing human CD16A or cynomolgus monkey CD16.

### Example 2: Preparation of the anti-human CD16A hybridoma monoclonal antibody

In the present disclosure, the anti-human CD16A monoclonal antibody is generated by immunizing mice. C57BL/6 mice, female, 6 weeks old (Jiangsu GemPharmatech Co., Ltd) were used in the experiment. The immune antigen was human CD16A extracellular segment protein (ACRO, CDA-H5220). For initial immunization, the antigen was mixed and emulsified with Freund's complete adjuvant (Sigma, F5881) for an intraperitoneal immunization, and each mouse was intraperitoneally injected with 100 µg. For subsequent immunization, the antigen was mixed the Ribi Adjuvant System (Sigma, S6322) for an intraperitoneal immunization. After the initial immunization, the immunization was performed every two weeks for a total of three times. The serum of the immunized mice was taken for an ELISA detection of antibody titers using conventional methods in the field.

Based on a detection result of the antibody titers, mice having high antibody titers in their serum were selected for the spleen cell fusion. A booster immunization was given to the selected mice 72 hours before the fusion using a mixture of the Ribi Adjuvant System and the above antigen through an intraperitoneal injection. The spleen lymphocytes were fused with Sp2/0 myeloma cells (ATCC, CRL-8287) by using an optimized PEG-mediated fusion procedure to obtain hybridoma cells. The fused hybridoma cells were resuspended in HAT complete medium (RPMI-1640 medium containing 20% FBS, 1×HAT, and 1×OPI), dispensed into a 96-well cell culture plate, and incubated at 37°C, 5% CO₂. On day 5 after the fusion, 50 µL of HAT complete medium was added to each well. On day 7 to day 8 after the fusion, based on a cell growth density, the medium was fully replaced with HT complete medium (RPMI-1640 medium containing 20% FBS, 1×HT, and 1×OPI) with 200 µL per well.

On day 10 to day 11 after the fusion, based on the cell growth density, a flow cytometry assay binding detection was performed. The medium of the positive wells were replaced with fresh medium. The cells were timely expanded to a 24-well plate based on a cell density. The cell lines transferred into the 24-well plate were retested, and then preserved and subcloned for the first round. The cells that were positive in the first round of subcloning were preserved and subcloned for the second round. The cells that were positive in the second round of subcloning were preserved and used for protein expression. The operations of subcloning and preservation are conventional technical operations in the field. The antibody was further prepared using a serum-free cell culture method. The murine antibody was purified by protein G affinity chromatography for a subsequent functional activity detection.

### Example 3: Murine CD16A antibody ELISA binding experiment

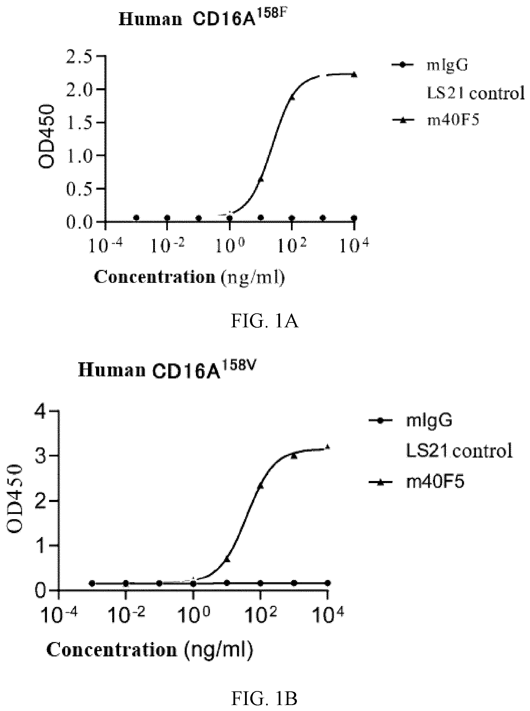

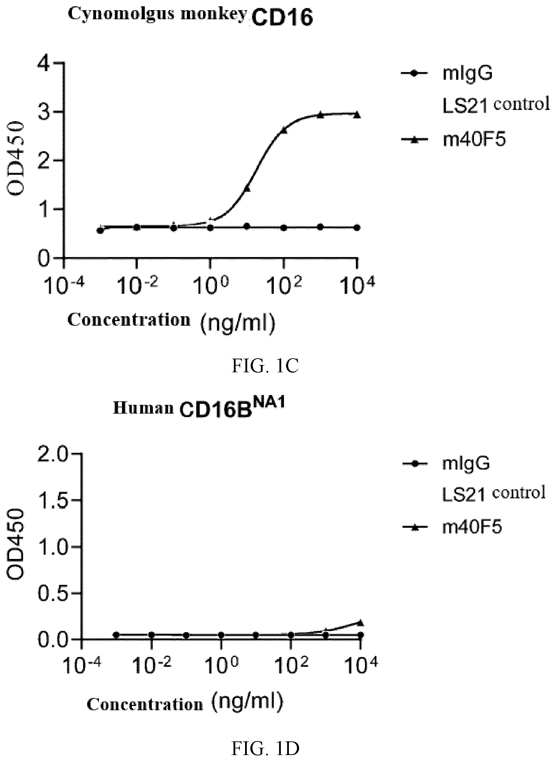

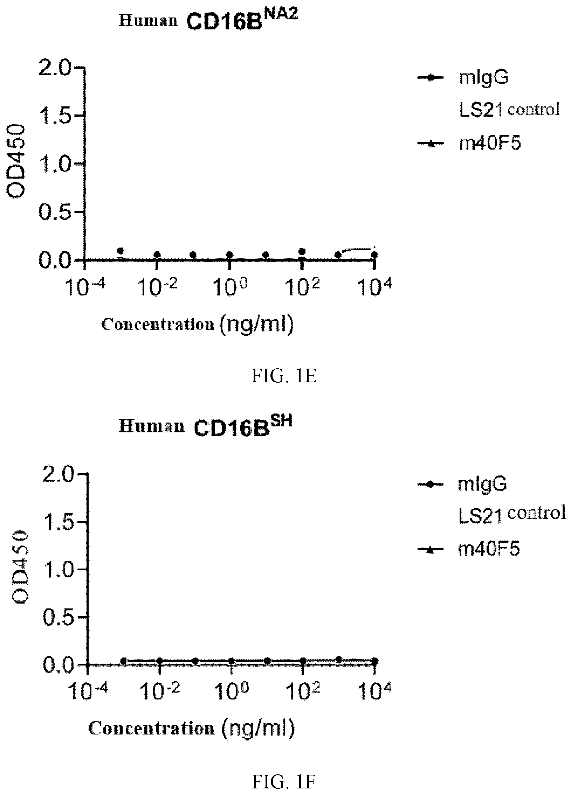

The ELISA experiment was performed to detect the binding properties of the murine CD16A antibody m40F5 obtained in Example 1. Each of human CD16A^{158F} (ACRO biosystems, CDA-H5220), human CD16A^{158V} (ACRO biosystems, CD8-H52H4), cynomolgus monkey CD16 (ACRO biosystems, FC6-C52H9), human CD16B^{NA1} (ACRO biosystems, CDB-H5227), human CD16B^{NA2} (ACRO biosystems, CDB-H5222), and human CD16B^{SH} (Sino Biological, 11046-H08H2) was diluted to 2 µg/mL with a coating buffer (35 mM NaHCO₃, 15 mM Na₂CO₃, pH 9.6), and 100 µL of the diluted antibody was added to each well of the enzyme-linked plate and left overnight at 4°C. Then, the plate was then washed three times with PBST (0.05% Tween 20-PBS, pH 7.2). 300 µL of blocking buffer (1% BSA, 0.05% Tween20-PBS, pH 7.2) was added to the plate and left to stand at room temperature for 2 hours. Then, the plate was washed again three times with PBST. Murine antibody m40F5 or control antibody LS21 (WO2006/125668 EN) was added to each well and incubated at room temperature for 1 hour. Again, the plate was washed three times with PBST. 100 µL of HRP-goat anti-mouse IgG secondary antibody (boster, Catalog No. BA1051) diluted with the blocking buffer was added to each well and incubated at room temperature for 1 hour. The plate was washed three times with PBST. TMB was added to each well. A reaction was performed at room temperature in the dark for 2 min to 5 min. The reaction was terminated with 2 M sulfuric acid. OD450 values were read using a microplate reader. FIG. 1A shows that the murine m40F5 antibody of the present disclosure can bind to human CD16A^{158F}. FIG. 1B shows that the murine m40F5 antibody of the present disclosure can also bind to human CD16A^{158V}. FIG. 1C shows that the murine m40F5 of the present disclosure can bind to cynomolgus monkey CD16. FIG. 1D shows that the murine m40F5 of the present disclosure does not bind to human CD16B^{NA1}. FIG. 1E shows that the murine 40F5 of the present disclosure does not bind to human CD16B^{NA2}. FIG. 1F shows that the murine 40F5 of the present disclosure does not bind to human CD16B^{SH}.

### Example 4: Flow cytometry assay binding experiment of murine CD16A antibody

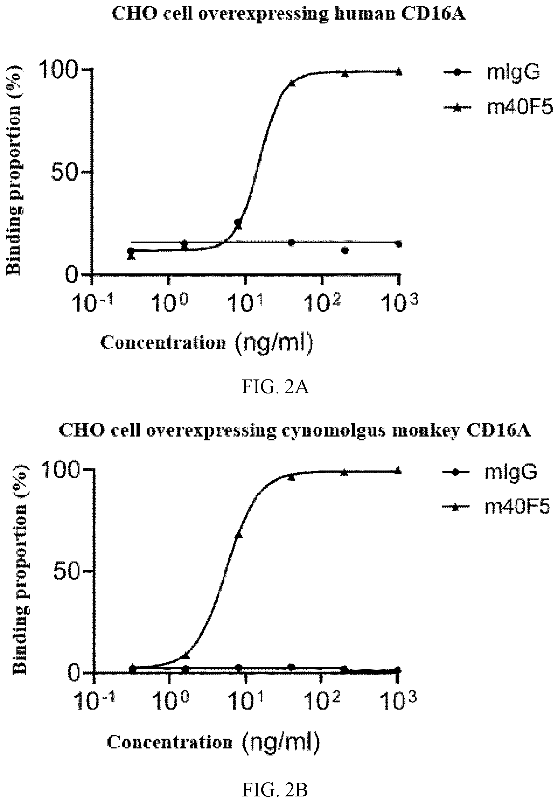

The CHO-K1 cells overexpressing human CD16A or CHO-K1 cells overexpressing cynomolgus monkey CD16 obtained in Example 1 were diluted to 2×10⁶/mL with PBS and added to a 1.5 mL EP tube at a volume of 100 µL/tube. Then, 10 µL of goat serum was added to each tube. The mixture was blocked at 4°C for 30 min. The CD16A antibody and the control antibody mIgG at gradient concentrations (5-fold serial dilution, with the highest final concentration of 10 µg/mL) were added and incubated at 4°C for 30 minutes. Each EP tube was filled with 1 mL of PBS and centrifuged at 3,500 rpm for 5 min at 4°C. The supernatant was completely discarded, and the cells were washed once again with PBS. After another centrifugation, the supernatant was completely discarded. The cells were resuspended in 100 µL/tube of PBS. Alexa-647-conjugated goat anti-murine antibody secondary antibody (Invitrogen) was added at 0.1 µL/tube. The cells were incubated at 4°C in the dark for 30 min, and were washed twice with PBS and centrifuged. Then, the supernatant was completely discarded. The cells were resuspended in 200 µL/tube of PBS and detected using a flow cytometer. The Results in FIG. 2A and FIG. 2B indicate that the murine m40F5 antibody can bind to CHO-K1 cells overexpressing human CD16A and CHO-K1 cells overexpressing cynomolgus monkey CD16, demonstrating that the murine CD16A antibody can bind to the CD16A protein on the cell surface.

### Example 5: Hybridoma cell sequencing

The total number of candidate hybridoma cells of the m40F5 antibody, selected in the above example, were cultured to 10⁶. The cells were collected by centrifugation at 800 rpm for 10 min and total RNA was extracted using the Trizol kit (Invitrogen). Using the total RNA as a template, a cDNA library was synthesized through reverse transcription (Invitrogen). The cDNA was served as a template for PCR amplification to obtain the nucleotide sequences of the variable regions of the m40F5 antibody corresponding to the hybridoma cells. The primer sequences used in the PCR amplification were complementary to the first framework region or signal peptide region and the constant region of the antibody variable region (specific sequence selection refers to Larrick, J.W., et al., (1990) Scand. J. Immunol., 32, 121-128 and Coloma, J.J. et al., (1991) BioTechniques, 11, 152-156). The PCR amplification was performed in a 50 µL reaction system containing 2 µL of cDNA, 5 µL of 10×PCR buffer, 2 µL (5 µM) of the upstream and downstream primers, 2 µL of dNTP, 1 µL of Taq enzyme (Takara, Ex Taq), and 38 µL of H₂O. Initial denaturation was performed at 95°C for 5 minutes, followed by temperature cycling for PCR amplification. The reaction conditions were: denaturation at 94°C for 30 S, annealing at 58°C for 45 S, and extension at 72°C for 50 S, for a total of 32 cycles, followed by extension at 72°C for 7 min. The amplified products were sequenced to obtain the sequence of the heavy chain variable region (SEQ ID NO: 23) and the sequence of the light chain variable region (SEQ ID NO: 24) of the murine monoclonal antibody m40F5.

### Example 6: Humanization of anti-human CD16A monoclonal antibody

### 6.1 Selection and back mutation of variable region frameworks of CD16A monoclonal antibody

On a basis of the results obtained in Example 5, by comparing the the IMGT human antibody heavy and light chain variable regions germline gene databases and using MOE software, the heavy chain or light chain variable region germline genes having high homology to the murine monoclonal antibody m40F5 were selected as templates. The CDRs of the murine monoclonal antibody were grafted onto the corresponding human templates to generate variable region sequences in the order of FR1-CDR1-FR2-CDR2-FR3-CDR3-FR4. The amino acid residues were identified and annotated based on the Kabat numbering system.

To maintain the conformation of the CDR region, back mutations were performed on the residues on the binding interface of VL and VH, the residues close to the CDRs and buried within the protein, and the residues that have direct interactions with the CDRs to ensure that the activity of the variable regions would not be affected.

### 6.2: Expression of humanized CD16A antibody

After the selection and the back mutation of the variable region frameworks of the CD16A monoclonal antibody, the heavy chain variable region sequence of the humanized CD16A antibody h40F5 obtained is shown as SEQ ID NO: 25, and the light chain variable region sequence of the humanized CD16A antibody h40F5 obtained is shown as SEQ ID NO: 26. The nucleotide sequences encoding the humanized antibody heavy chain (SEQ ID NO: 39) and the nucleotide sequences encoding the humanized antibody light chain (SEQ ID NO: 40) were recombined into pTT5 plasmid respectively by using molecular biology techniques.

The CD16A antibody was prepared by transiently transfecting ExpiCHO-S cells (Gibco, Catalog No. A29127) with pTT5 vectors carrying light chains and heavy chains of the humanized anti-human CD16A antibody h40F5. One day before transfection, a cell density of ExpiCHO-S cells was adjusted to (3~4)×10⁶/mL and cultured overnight at 37°C and 8% CO₂, with shaking at 120 rpm. On the day of transfection, when the cell density reached 7×10⁶/mL to 1×10⁷/mL and the viability exceeded 95%, the cells were ready for transfection. The cells were diluted to 6×10⁶/mL using fresh pre-warmed ExpiCHO medium (Gibco, Catalog No. A2910002). The pTT5 plasmids carrying the light and heavy chains of the humanized anti-human CD16A antibody h40F5 and the ExpiFectamine CHO transfection reagent (Gibco, Catalog No. A29129) were transfected into ExpiCHO-S cells at a molar ratio of 2:1 and cultured at 37°C and 8% CO₂, with shaking at 120 rpm. 18 h to 22h after transfection, ExpiFectamine CHO Enhancer and ExpiCHO Feed were mixed well and immediately added to the transfected cells, which were then mixed well and cultured at 32°C and 5% CO₂, with shaking at 120 rpm. On the fifth day after transfection, an additional 8 mL of ExpiCHO Feed was added to the cells, mixed well, and continued to be cultured. Changes of cell count and viability were observed every day. Cells were harvested by centrifugation when the cell viability dropped below 80% or after 10 days to 14 days of culture. The expressed supernatant was filtered through a 0.22 µm filter. The antibodies containing the Fc domain were captured from the expressed supernatant using a Mabselect prism A affinity chromatography column (GE Healthcare, Catalog No. 17549854). After equilibrating the chromatography column with phosphate buffer at pH 7.2, the supernatant was passed through the affinity chromatography column and eluted with an elution buffer (100 mM citric acid, pH 2.7). Finally, the antibody was concentrated and replaced with PBS buffer. The purity of the purified antibody was identified to be over 95% by SDS-PAGE, and finally the humanized h40F5 antibody (heavy chain as shown in SEQ ID NO: 35 and light chain as shown in SEQ ID NO: 36) was obtained.

### 6.3 Affinity verification of humanized antibody h40F5

The affinity of the humanized h40F5 antibody to each of the human CD16A antigen, the human CD16B antigen, and the monkey CD16 antigen was tested by BIACORE. The results are shown in Table 2. The humanized antibody obtained has a good affinity to the CD16A antigen.

**Table 2**

| Antibody | Antigen | **ka (1/Ms)** | **kd (1/s)** | **KD (M)** |
|---|---|---|---|---|
| h40F5 | Human CD16A | 8.31E+05 | 9.60E-04 | 1.15E-09 |
| h40F5 | Cynomolgus monkey CD16 | 1.32E+06 | 3.90E-04 | 2.96E-10 |
| h40F5 | Human CD16B | NA | NA | NA |

### Example 7: ELISA binding experiment for humanized antibody h40F5

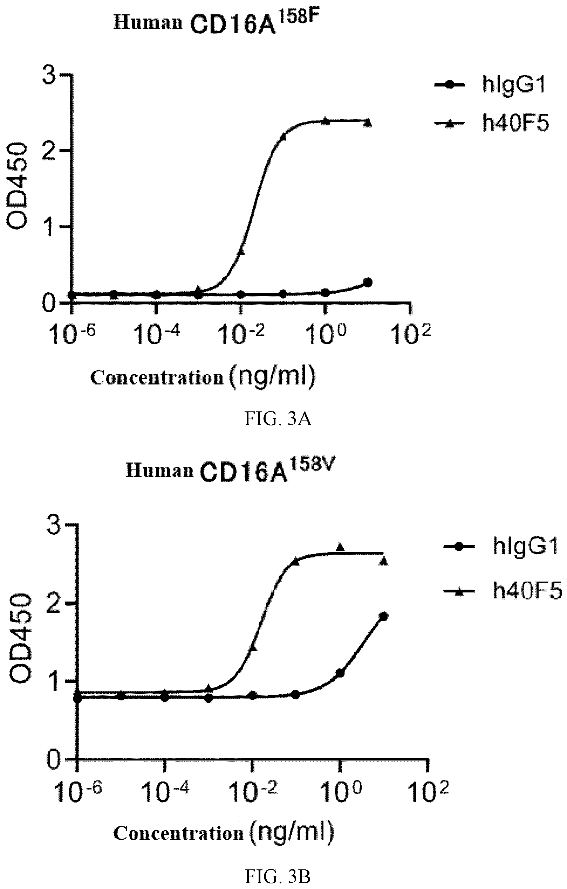

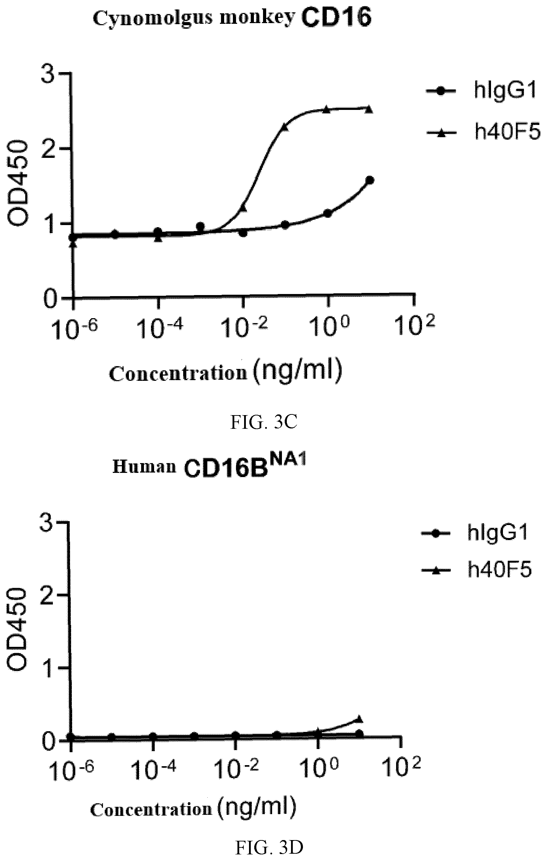

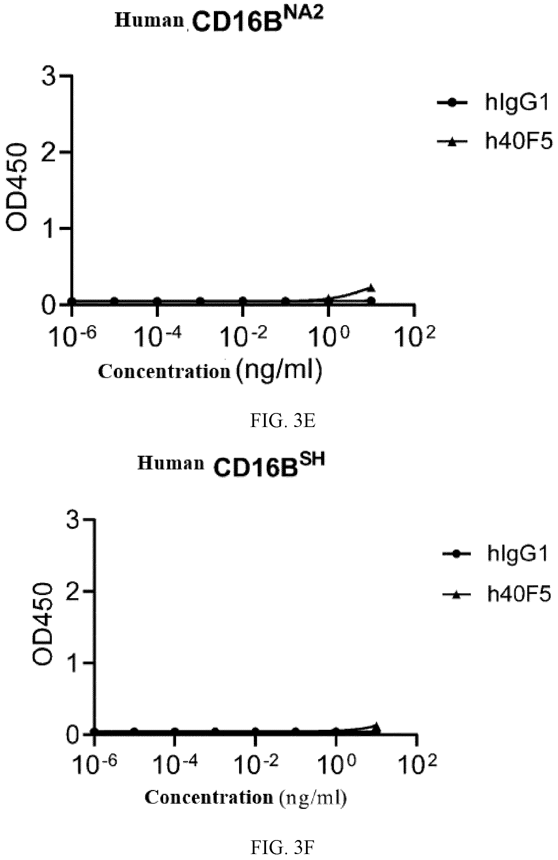

The ELISA experiment was performed to detect the binding properties of the humanized antibody h40F5, with the wild-type human IgG1 antibody as a control. Each of human CD16A^{158F} (ACRO biosystems, CDA-H5220), human CD16A^{158V} (ACRO biosystems, CD8-H52H4), cynomolgus monkey CD16 (ACRO biosystems, FC6-C52H9), human CD16B^{NA1} (ACRO biosystems, CDB-H5227), human CD16B^{NA2} (ACRO biosystems, CDB-H5222), and human CD16B^{SH}(Sino Biological, 11046-H08H2) was diluted to 2 µg/mL with the coating buffer (35 mM NaHCO₃, 15 mM Na₂CO₃, pH 9.6). 100 µL of the diluted solution was added to each well of the enzyme-linked plate and incubated overnight at 4°C. Then, the plate was washed three times with PBST (0.05% Tween 20-PBS, pH 7.2). Then, 300 µL of blocking buffer (1% BSA, 0.05% Tween20-PBS, pH 7.2) was added to the plate and left to stand at room temperature for 2 hours. The plate was washed three times with PBST again. The humanized anti-human CD16A antibody h40F5 or the human IgG1 antibody at gradient concentrations was added to each well and incubated at room temperature for 1 hour. Again, the plate was washed three times with PBST. 100 µL of HRP-goat anti-human IgG secondary antibody (Jackson ImmunoResearch, 109-036-170) diluted with the blocking buffer was added to each well and incubated at room temperature for 1 hour. The plate was washed three times with PBST. TMB was added to each well. A reaction was performed at room temperature in the dark for 2 min to 5 min. The reaction was terminated by adding 2 M sulfuric acid to each well. OD450 values were read using a microplate reader. FIG. 3A shows that the humanized h40F5 antibody of the present disclosure can bind to human CD16A^{158F}. FIG. 3B shows that the humanized h40F5 antibody of the present disclosure can also bind to human CD16A^{158V}. FIG. 3C shows that the humanized h40F5 of the present disclosure can bind to cynomolgus monkey CD16. FIG. 3D shows that the humanized h40F5 of the present disclosure does not bind to human CD16B^{NA1}. FIG. 3E shows that the humanized h40F5 of the present disclosure does not bind to human CD16B^{NA2}. FIG. 3F shows that the humanized h40F5 of the present disclosure does not bind to human CD16B^{SH}.

### Example 8: Preparation of the CD16A×BCMA bispecific antibody and detection of binding activity and cell killing ability

Based on the experimental results of Example 1 to Example 7, the bispecific antibody CD16A×BCMA was designed and the binding activity and the cell killing ability of the bispecific antibody were tested by the inventors.

### 8.1: Design and preparation of the CD16A×BCMA bispecific antibody

The antibody contains two monovalent units. One of the two monovalent units is in the form of anti-CD16A scFv-Fc, with amino acid sequences of its heavy chain variable region and light chain variable region derived from the sequence of the humanized antibody h40F5 obtained in Example 6 of the present disclosure. The other one of the two monovalent units is in the form of anti-BCMA scFv-Fc (the sequence of which was from US009273141B2). This bispecific antibody is named CD16A×BCMA. The bispecific antibody consists of two polypeptide chains, namely: a heavy chain containing a CD16A single-chain antibody scFv (SEQ ID NO: 41), and a heavy chain containing a BCMA single-chain antibody scFv (SEQ ID NO: 42), with the heavy chain constant regions of the two chains derived from the human antibody IgG1. Due to the special asymmetric structure of the molecule, in order to reduce the formation of homodimers, different amino acid mutations were introduced into the constant regions of the two chains. Meanwhile, in order to prevent cross-linking activation caused by Fcγ receptors, (L234A/L235A) mutations were also introduced into the heavy chain constant regions.

The bispecific antibody was prepared by the method described in reference to Example 6.2, in combination with a plasmid ratio (1:1 or other ratios) and through one-step affinity purification. The relevant sequences of the bispecific antibody CD16A×BCMA are shown in Table 3.

**Table 3**

| | CD16 heavy chain | BCMA heavy chain |
|---|---|---|
| Amino acid sequence | SEQ ID NO: 47 | SEQ ID NO: 48 |
| Nucleotide sequence | SEQ ID NO: 54 | SEQ ID NO: 55 |

### 8.2: Measurement of binding activity of the CD16A×BCMA bispecific antibody to the antigen (ELISA)

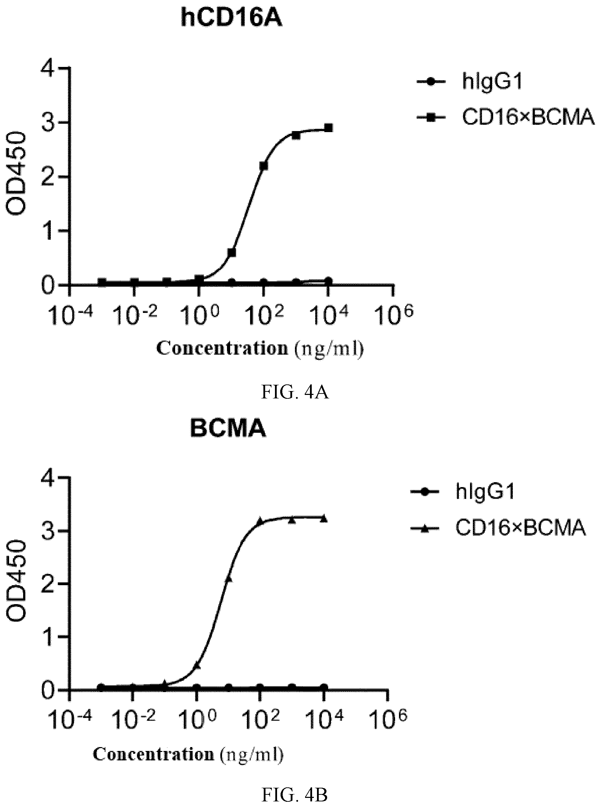

Human BCMA (ACRO biosystems, BCA-H522y) or human CD16A antigen (ACRO biosystems, CDD-H52W1) was diluted to 2 µg/mL with the coating buffer (35 mM NaHCO₃, 15 mM Na₂CO₃, pH 9.6). 100 µL of the diluted solution was added to each well of the enzyme-linked plate and incubated overnight at 4°C. Then, the plate was washed three times with PBST (0.05% Tween 20-PBS, pH 7.2). 300 µL of blocking buffer (1% BSA, 0.05% Tween20-PBS, pH 7.2) was added to the plate and left to stand at room temperature for 2 hours. The plate was washed three times with PBST again. The corresponding bispecific antibody or the control antibody hIgG1 was added to each well and incubated at room temperature for 1 hour. The precipitate was washed three times with PBST. 100 µL of HRP-goat anti-human IgG secondary antibody (Jackson ImmunoResearch, 109-036-170) diluted with the blocking buffer was added to each well and incubated at room temperature for 1 hour. The plate was washed three times with PBST. TMB was added to each well. A reaction was performed at room temperature in the dark for 2 min to 5 min. The reaction was terminated by adding 2 M sulfuric acid to each well. OD450 values were read using a microplate reader. FIG. 4A and FIG. 4B show that the CD16A×BCMA of the present disclosure can bind to both CD16A and BCMA antigens.

### 8.3 Cell killing assay

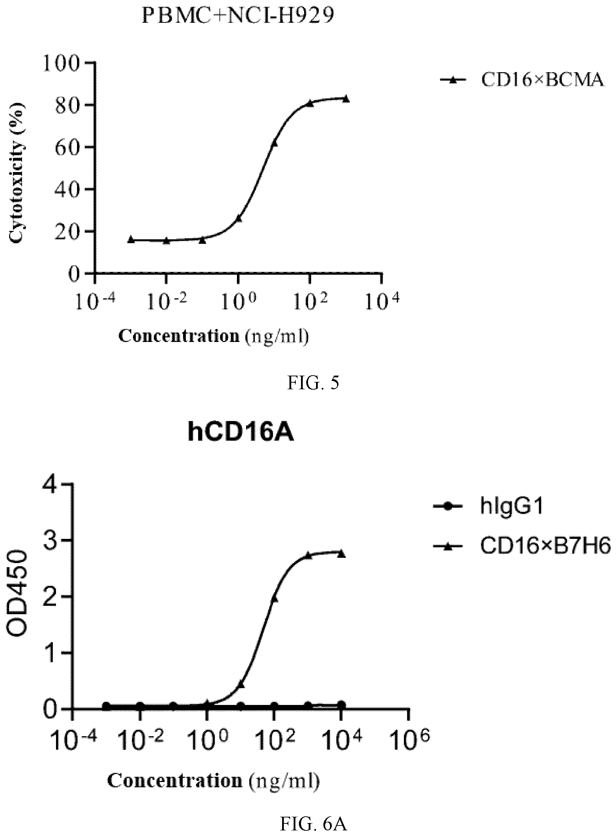

NCI-H929 multiple myeloma cells were labeled with CellTrace Violet (thermo, C34557). The cell density was adjusted to 1×10⁵/mL. The cell density of PBMCs was adjusted to 1×10⁶/mL. The PBMCs and the labeled NCI-H929 cells were mixed at a ratio of 1:1 and added to a 96-well round-bottom plate. Then, the above bispecific antibody at gradient concentrations were added and mixed evenly. After incubation at 37°C for 24 hours, the cells were filtered through a 200-mesh nylon net into flow cytometry tubes, and 7-AAD was added, 10 minutes after 7-AAD was added, a detection was performed. The proportion of CTV+7AAD+ cells was used to characterize the killing efficiency. FIG. 5 shows that as the concentration of the CD16A×BCMA bispecific antibody increases, the killing efficiency of PBMCs against NCI-H929 multiple myeloma cells gradually increases.

### Example 9: Preparation of the CD16A×B7H6 bispecific antibody and detection of binding activity and cell killing ability

Based on the experimental results of Example 1 to Example 6, the bispecific antibody CD16A×B7H6 was designed and the binding activity and the cell killing ability of the bispecific antibody were tested by the inventors.

### 9.1: Design and preparation of the CD16A×B7H6 bispecific antibody

The antibody contains two monovalent units. One of the two monovalent units is in the form of anti-CD16A scFv-Fc, in which the light chain variable region and the heavy chain variable region of CD16A scFv are derived from the humanized sequence obtained in Example 6 of the present disclosure. The other one of the two monovalent units is in the form of anti-B7H6 scFv-Fc (the B7H6 scFv sequence was from CN114395045A). This bispecific antibody is named CD16A×B7H6. This bispecific antibody consists of two polypeptide chains: a heavy chain containing the CD16A single-chain antibody scFv (SEQ ID NO: 41), and a heavy chain containing the B7H6 single-chain antibody scFv (SEQ ID NO: 43). Due to the special asymmetric structure of the molecule, in order to reduce the formation of homodimers, different amino acid mutations were introduced into the constant regions of the two chains. Meanwhile, in order to prevent cross-linking activation caused by Fcγ receptors, (L234A/L235A) mutations were also introduced into the heavy chain constant regions.

The bispecific antibody CD16A×B7H6 was prepared by the method described in reference to Example 6.2, in combination with a plasmid ratio (1: 1 or other ratios) and the bispecific antibody CD16A×B7H6 was prepared through one-step affinity purification. The relevant sequences of the bispecific antibody CD16A×B7H6 are shown in Table 4.

**Table 4**

| Item | CD16A heavy chain | B7H6 heavy chain |
|---|---|---|
| Amino acid sequence | SEQ ID NO: 47 | SEQ ID NO: 49 |
| Nucleotide sequence | SEQ ID NO: 54 | SEQ ID NO: 56 |

### 9.2: Determination of binding activity of the bispecific antibody to the antigen (ELISA)

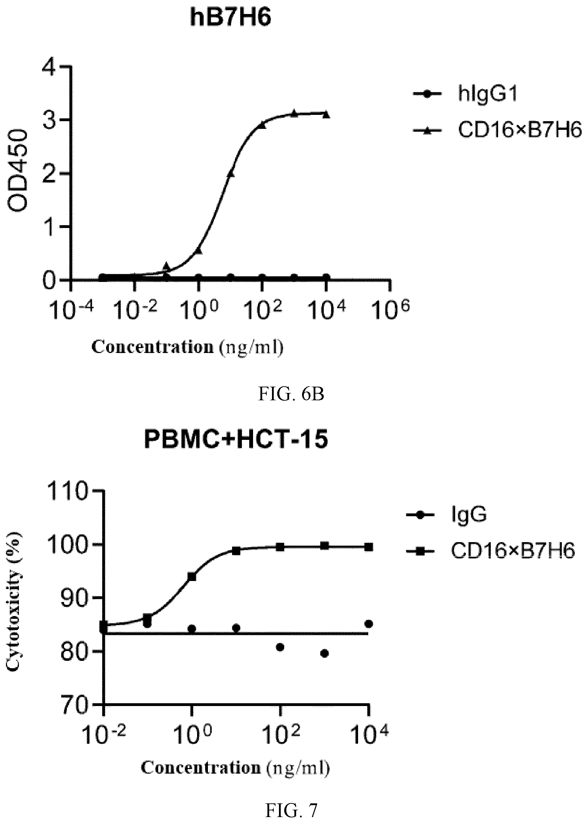

Human B7H6 (ACRO biosystems, B76-H52H8) or human CD16A antigen (ACRO biosystems, Catalog No. CDA-H5220) was diluted to 2 µg/mL with the coating buffer (35 mM NaHCO₃, 15 mM Na₂CO₃, pH 9.6). 100 µL of the diluted solution was added to each well of the enzyme-linked plate and incubated overnight at 4°C. Then, the plate was washed three times with PBST (0.05% Tween 20-PBS, pH 7.2). 300 µL of blocking buffer (1% BSA, 0.05% Tween20-PBS, pH 7.2) was added to the plate and left to stand at room temperature for 2 hours. The plate was washed three times with PBST again. The corresponding bispecific antibody or control antibody hIgG was added to each well and incubated at room temperature for 1 hour. Again, the plate was washed three times with PBST. 100 µL of HRP-goat anti-human IgG secondary antibody (Jackson ImmunoResearch, 109-036-170) diluted with the blocking buffer was added to each well and incubated at room temperature for 1 hour. After incubation, the precipitate was washed three times with PBST. TMB was added to each well. A reaction was performed at room temperature in the dark for 2 min to 5 min. The reaction was terminated by adding 2 M sulfuric acid to each well. OD450 values were read using a microplate reader. FIG. 6A and FIG. 6B show that the bispecific antibody CD16A×B7H6 of the present disclosure can effectively bind to both CD16A and B7H6 antigens.

### 9.3 Cell killing assay

Colorectal cancer HCT-15 cells were digested and counted, and the cell density was adjusted to 2×10⁵/mL. The RTCA instrument (Agilent) was turned on to select the instrument type DP and experimental mode, and to fill in cell information and medicament. After entering the "schedule" set-up experiment step, 50 µL of fresh medium (89% RPMI 1640 medium + 10% fetal bovine serum + 1% penicillin-streptomycin) was added to the plate, and the plate was placed into the instrument. The instrument was then closed, and the first step was initiated by clicking. The plate was taken out after the first step was "Done", added with 100 µL of cell suspension and left to stand at room temperature for 15 min to 30 min to prevent edge effect. Then, the plate was placed back into the instrument, and "Start" button was clicked. After the cells grew to the logarithmic phase, the assay was paused to add 50 µL of PBMCs (4×10⁶/mL) and the bispecific antibody CD16A×B7H6 or control antibody hIgG at gradient concentrations. "Start" button was clicked. An analysis was performed after a period of time. The result in FIG. 7 shows that as the concentration of the CD16A×B7H6 bispecific antibody increases, the killing efficiency of PBMCs on colorectal cancer HCT-15 cells gradually increases, indicating that CD16A×B7H6 can effectively promote PBMCs to specifically kill B7H6⁺ tumor cells.

In the description of this specification, the descriptions referring the terms "one embodiment", "some embodiments", "example", "specific examples", or "some examples", etc., mean that a specific feature, structure, material, or characteristic described in connection with the embodiment or example is included in at least one embodiment or example of the present disclosure. In this specification, schematic representations of the terms above do not necessarily refer to the same embodiment or example. Furthermore, the specific features, structures, materials, or characteristics described may be combined in any suitable manner in any one or more embodiments or examples. Furthermore, the different embodiments or examples and the features of the different embodiments or examples described in this specification can be integrated and combined by those skilled in the art without contradicting each other.

While embodiments of the present disclosure have been illustrated and described above, it will be understood that the above-described embodiments are illustrative and should not be construed as limiting the present disclosure, and changes, modifications, substitutions, and alterations may be made to the above-mentioned embodiments by those skilled in the art without departing from the scope of the present disclosure.

## Claims

1. An antibody or antigen-binding fragment, comprising:
a heavy chain variable region CDR1 sequence, a heavy chain variable region CDR2 sequence, and a heavy chain variable region CDR3 sequence as set forth in SEQ ID NO: 1, SEQ ID NO: 2, and SEQ ID NO: 3, respectively, or as set forth in amino acid sequences having at least 95% identity to SEQ ID NO: 1, SEQ ID NO: 2, and SEQ ID NO: 3, respectively; and/or
a light chain variable region CDR1 sequence, a light chain variable region CDR2 sequence, and a light chain variable region CDR3 sequence as set forth in SEQ ID NO: 4, SEQ ID NO: 5, and SEQ ID NO: 6, respectively, or as set forth in amino acid sequences having at least 95% identity to SEQ ID NO: 4, SEQ ID NO: 5, and SEQ ID NO: 6, respectively.

2. The antibody or antigen-binding fragment according to claim 1, comprising:
the heavy chain variable region CDR1 sequence as set forth in SEQ ID NO: 1;
the heavy chain variable region CDR2 sequence as set forth in SEQ ID NO: 2;
the heavy chain variable region CDR3 sequence as set forth in SEQ ID NO: 3;
the light chain variable region CDR1 sequence as set forth in SEQ ID NO: 4;
the light chain variable region CDR2 sequence as set forth in SEQ ID NO: 5; and
the light chain variable region CDR3 sequence as set forth in SEQ ID NO: 6.

3. The antibody or antigen-binding fragment according to claim 2, comprising:
at least one of a heavy chain framework region, FR, and a light chain FR.

4. The antibody or antigen-binding fragment according to claim 3, wherein at least part of the at least one of the heavy chain FR and the light chain FR is derived from at least one of a humanized antibody, a primate antibody, a murine antibody, or a mutant thereof.

5. The antibody or antigen-binding fragment according to claim 4, comprising:
at least one of a heavy chain framework region HFR1 sequence, a heavy chain framework region HFR2 sequence, a heavy chain framework region HFR3 sequence, and a heavy chain framework region HFR4 sequence as set forth in SEQ ID NO: 7 to SEQ ID NO: 10, respectively; or
at least one of a heavy chain framework region HFR1 sequence, a heavy chain framework region HFR2 sequence, a heavy chain framework region HFR3 sequence, and a heavy chain framework region HFR4 sequence as set forth in SEQ ID NO: 15 to SEQ ID NO: 18, respectively.

6. The antibody or antigen-binding fragment according to claim 4, comprising:
at least one of a light chain framework region LFR1 sequence, a light chain framework region LFR2 sequence, a light chain framework region LFR3 sequence, and a light chain framework region LFR4 sequence as set forth in SEQ ID NO: 11 to SEQ ID NO: 14, respectively; or
at least one of a light chain framework region LFR1 sequence, a light chain framework region LFR2 sequence, a light chain framework region LFR3 sequence, and a light chain framework region LFR4 sequence as set forth in SEQ ID NO: 19 to SEQ ID NO: 22, respectively.

7. The antibody or antigen-binding fragment according to any one of claims 5 to 6, comprising:
at least one of the heavy chain framework region HFR1 sequence, the heavy chain framework region HFR2 sequence, the heavy chain framework region HFR3 sequence, and the heavy chain framework region HFR4 sequence as set forth in SEQ ID NO: 7 to SEQ ID NO: 10, respectively, and at least one of the light chain framework region LFR1 sequence, the light chain framework region LFR2 sequence, the light chain framework region LFR3 sequence, and the light chain framework region LFR4 sequence as set forth in SEQ ID NO: 11 to SEQ ID NO: 14, respectively; or
at least one of the heavy chain framework region HFR1 sequence, the heavy chain framework region HFR2 sequence, the heavy chain framework region HFR3 sequence, and the heavy chain framework region HFR4 sequence as set forth in SEQ ID NO: 15 to SEQ ID NO: 18, respectively, and at least one of the light chain framework region LFR1 sequence, the light chain framework region LFR2 sequence, the light chain framework region LFR3 sequence, and the light chain framework region LFR4 sequence as set forth in SEQ ID NO: 19 to SEQ ID NO: 22, respectively.

8. The antibody or antigen-binding fragment according to claim 7, comprising:
a heavy chain variable region as set forth in SEQ ID NO: 23 or SEQ ID NO: 25; and/or
a light chain variable region as set forth in SEQ ID NO: 24 or SEQ ID NO: 26.

9. The antibody or antigen-binding fragment according to claim 8, comprising:
1) the heavy chain variable region as set forth in SEQ ID NO: 23 and the light chain variable region as set forth in SEQ ID NO: 24; or
2) the heavy chain variable region as set forth in SEQ ID NO: 25 and the light chain variable region as set forth in SEQ ID NO: 26.

10. The antibody or antigen-binding fragment according to any one of claims 1 to 9, comprising:
at least one of a heavy chain constant region and a light chain constant region, wherein at least part of the at least one of the heavy chain constant region and the light chain constant region is derived from at least one of a humanized antibody, a primate antibody, a murine antibody, or a mutant thereof.

11. The antibody or antigen-binding fragment according to claim 10, wherein the light chain constant region and the heavy chain constant region are both derived from a murine IgG antibody or a mutant thereof, or a humanized IgG antibody or a mutant thereof.

12. The antibody or antigen-binding fragment according to claim 11, wherein the light chain constant region and the heavy chain constant region are both derived from a murine IgG1 antibody or a mutant thereof, or a human IgG1 antibody or a mutant thereof.

13. The antibody or antigen-binding fragment according to claim 12, wherein the antibody comprises:
a heavy chain constant region having an amino acid sequence as set forth in SEQ ID NO: 27 or SEQ ID NO: 29; and/or
a light chain constant region having an amino acid sequence as set forth in SEQ ID NO: 28 or SEQ ID NO: 30.

14. The antibody or antigen-binding fragment according to claim 10, wherein the antibody or antigen-binding fragment comprises:
a heavy chain having an amino acid sequence as set forth in any one of SEQ ID NO: 31, SEQ ID NO: 33, and SEQ ID NO: 35; and
a light chain having an amino acid sequence as set forth in any one of SEQ ID NO: 32, SEQ ID NO: 34, and SEQ ID NO: 36.

15. The antibody or antigen-binding fragment according to claim 10, wherein the antibody or antigen-binding fragment has:
a heavy chain having an amino acid sequence as set forth in SEQ ID NO: 31 and a light chain having an amino acid sequence as set forth in SEQ ID NO: 32;
a heavy chain having an amino acid sequence as set forth in SEQ ID NO: 33 and a light chain having an amino acid sequence as set forth in SEQ ID NO: 34; or
a heavy chain having an amino acid sequence as set forth in SEQ ID NO: 35 and a light chain having an amino acid sequence as set forth in SEQ ID NO: 36.

16. The antibody or antigen-binding fragment according to claim 1, wherein the antibody or antigen-binding fragment comprises a monoclonal antibody or a polyclonal antibody.

17. The antibody or antigen-binding fragment according to claim 16, wherein the monoclonal antibody comprises at least one of a full-length antibody, Fv, a single-chain antibody, Fab, a single-domain antibody, and a minimal recognition unit.

18. The antibody or antigen-binding fragment according to claim 16, wherein the antibody or antigen-binding fragment is capable of binding to an amino acid sequence as set forth in SEQ ID NO: 37 and/or SEQ ID NO: 38.

19. A bispecific binding molecule, comprising:
a first binding region comprising the antibody or antigen-binding fragment according to any one of claims 1 to 18; and
a second binding region having a binding activity to BCMA or B7H6.

20. The bispecific binding molecule according to claim 19, wherein the bispecific binding molecule comprises a symmetric bispecific binding molecule or an asymmetric bispecific binding molecule.

21. The bispecific binding molecule according to claim 19, wherein the bispecific binding molecule is an asymmetric bispecific binding molecule.

22. The bispecific binding molecule according to claim 19, wherein the antibody or antigen-binding fragment is an anti-CD16A single-chain antibody.

23. The bispecific binding molecule according to claim 22, wherein the second binding region comprises at least one of a full-length antibody, Fv, a single-chain antibody, Fab, a single-domain antibody, and a minimal recognition unit that have a binding activity to BCMA or B7H6.

24. The bispecific binding molecule according to claim 23, wherein the second binding region comprises an anti-BCMA single-chain antibody or an anti-B7H6 single-chain antibody.

25. The bispecific binding molecule according to claim 24, wherein the anti-CD16A single-chain antibody comprises an anti-CD16A antibody light chain variable region and an anti-CD16A antibody heavy chain variable region, wherein:
the anti-CD16A antibody heavy chain variable region has an amino acid sequence as set forth in SEQ ID NO: 23 or SEQ ID NO: 25; and
the anti-CD16A antibody light chain variable region has an amino acid sequence as set forth in SEQ ID NO: 24 or SEQ ID NO: 26.

26. The bispecific binding molecule according to claim 25, wherein the anti-CD16A single-chain antibody further comprises a linker peptide 1, wherein:
an N-terminus of the linker peptide 1 is linked to a C-terminus of the anti-CD16A antibody heavy chain variable region, and a C-terminus of the linker peptide 1 is linked to an N-terminus of the anti-CD16A antibody light chain variable region; or
the N-terminus of the linker peptide 1 is linked to a C-terminus of the anti-CD16A antibody light chain variable region, and the C-terminus of the linker peptide 1 is linked to an N-terminus of the anti-CD16A antibody heavy chain variable region.

27. The bispecific binding molecule according to claim 26, wherein the anti-BCMA single-chain antibody comprises an anti-BCMA antibody light chain variable region and an anti-BCMA antibody heavy chain variable region, wherein:
the anti-BCMA antibody heavy chain variable region has an amino acid sequence as set forth in SEQ ID NO: 59; and
the anti-BCMA antibody light chain variable region has an amino acid sequence as set forth in SEQ ID NO: 60.

28. The bispecific binding molecule according to claim 27, wherein the anti-BCMA single-chain antibody further comprises a linker peptide 2, wherein:
an N-terminus of the linker peptide 2 is linked to a C-terminus of the anti-BCMA antibody heavy chain variable region, and a C-terminus of the linker peptide 2 is linked to an N-terminus of the anti-BCMA antibody light chain variable region; or
the N-terminus of the linker peptide 2 is linked to a C-terminus of the anti-BCMA antibody light chain variable region, and the C-terminus of the linker peptide 2 is linked to an N-terminus of the anti-BCMA antibody heavy chain variable region.

29. The bispecific binding molecule according to claim 28, wherein the anti-B7H6 single-chain antibody comprises an anti-B7H6 antibody light chain variable region and an anti-B7H6 antibody heavy chain variable region, wherein:
the anti-B7H6 antibody heavy chain variable region has an amino acid sequence as set forth in SEQ ID NO: 61, and
the anti-B7H6 antibody light chain variable region has an amino acid sequence as set forth in SEQ ID NO: 62.

30. The bispecific binding molecule according to claim 29, wherein the anti-B7H6 single-chain antibody further comprises a linker peptide 3, wherein:
an N-terminus of the linker peptide 3 is linked to a C-terminus of the anti-B7H6 antibody heavy chain variable region, and a C-terminus of the linker peptide 3 is linked to an N-terminus of the anti-B7H6 antibody light chain variable region; or
the N-terminus of the linker peptide 3 is linked to a C-terminus of the anti-B7H6 antibody light chain variable region, and the C-terminus of the linker peptide 3 is linked to an N-terminus of the anti-B7H6 antibody heavy chain variable region.

31. The bispecific binding molecule according to claim 30, wherein at least one of the linker peptide 1, the linker peptide 2, and the linker peptide 3 has an amino acid sequence set forth in (GGGGS)n, where n is an integer greater than or equal to 1.

32. The bispecific binding molecule according to claim 31, wherein n is 1, 2, 3, 4, 5, 6, 7, 8, 9, or 10.

33. The bispecific binding molecule according to claim 31, wherein at least one of the linker peptide 1, the linker peptide 2, and the linker peptide 3 has an amino acid sequence as set forth in SEQ ID NO: 44.

34. The bispecific binding molecule according to claim 22, wherein the anti-CD16A single-chain antibody has an amino acid sequence as set forth in SEQ ID NO: 41.

35. The bispecific binding molecule according to claim 24, wherein the anti-BCMA single-chain antibody has an amino acid sequence as set forth in SEQ ID NO: 42.

36. The bispecific binding molecule according to claim 24, wherein the anti-B7H6 single-chain antibody has an amino acid sequence as set forth in SEQ ID NO: 43.

37. The bispecific binding molecule according to claim 19, wherein the first binding region further comprises:
a first Fc peptide segment, an N-terminus of the first Fc peptide segment being linked to a C-terminus of the antibody or antigen-binding fragment.

38. The bispecific binding molecule according to claim 37, wherein the second binding region further comprises:
a second Fc peptide segment, an N-terminus of the second Fc peptide segment being linked to a C-terminus of the anti-BCMA single-chain antibody or the anti-B7H6 single-chain antibody.

39. The bispecific binding molecule according to claim 38, wherein the first Fc peptide segment has an amino acid sequence as set forth in SEQ ID NO: 45.

40. The bispecific binding molecule according to claim 39, wherein the second Fc peptide segment has an amino acid sequence as set forth in SEQ ID NO: 46.

41. The bispecific binding molecule according to claim 40, wherein the first Fc peptide segment and the second Fc peptide segment are linked by a knob-into-hole structure.

42. The bispecific binding molecule according to claim 19, wherein:
the first binding region has an amino acid sequence as set forth in SEQ ID NO: 47; and
the second binding region has an amino acid sequence as set forth in SEQ ID NO: 48 or SEQ ID NO: 49.

43. A nucleic acid molecule, encoding the antibody or antigen-binding fragment according to any one of claims 1 to 18, or the bispecific binding molecule according to any one of claims 19 to 42.

44. An expression vector, carrying the nucleic acid molecule according to claim 43.

45. A recombinant cell, carrying the nucleic acid molecule according to claim 43 or the expression vector according to claim 44, or being capable of expressing the antibody or antigen-binding fragment according to any one of claims 1 to 18 or the bispecific binding molecule according to any one of claims 19 to 42.

46. The recombinant cell according to claim 45, wherein the recombinant cell is obtained by introducing the expression vector according to claim 44 into a host cell.

47. The recombinant cell according to claim 46, wherein the recombinant cell is a eukaryotic cell.

48. The recombinant cell according to claim 46, wherein the recombinant cell is a mammalian cell.

49. A composition, comprising the antibody or antigen-binding fragment according to any one of claims 1 to 18, the bispecific binding molecule according to any one of claims 19 to 42, the nucleic acid molecule according to claim 43, the expression vector according to claim 44, or the recombinant cell according to any one of claims 45 to 48.

50. A medicament, comprising the antibody or antigen-binding fragment according to any one of claims 1 to 18, the bispecific binding molecule according to any one of claims 19 to 42, the nucleic acid molecule according to claim 43, the expression vector according to claim 44, the recombinant cell according to any one of claims 45 to 48, or the composition according to claim 49.

51. A kit, comprising the antibody or antigen-binding fragment according to any one of claims 1 to 18, the bispecific binding molecule according to any one of claims 19 to 42, the nucleic acid molecule according to claim 43, the expression vector according to claim 44, or the recombinant cell according to any one of claims 45 to 48.

52. Use of the antibody or antigen-binding fragment according to any one of claims 1 to 18, the nucleic acid molecule according to claim 43, the expression vector according to claim 44, the recombinant cell according to any one of claims 45 to 48, or the composition according to claim 49 in the preparation of a medicament for preventing and/or treating a CD16A-mediated disease.

53. The use according to claim 52, wherein the CD16A-mediated disease comprises an autoimmune disease.

54. The use according to claim 52, wherein the autoimmune disease comprises:
at least one of systemic lupus erythematosus, rheumatoid arthritis, systemic vasculitis, scleroderma, dermatomyositis, autoimmune hemolytic anemia, thyroid autoimmune disease, ulcerative colitis, chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritis syndrome, pemphigus vulgaris, pemphigoid, primary biliary cirrhosis, multiple sclerosis, and acute idiopathic polyneuritis.

55. Use of the bispecific binding molecule according to any one of claims 19 to 42, the nucleic acid molecule according to claim 43, the expression vector according to claim 44, the recombinant cell according to any one of claims 45 to 48, or the composition according to claim 49 in the preparation of a medicament for preventing and/or treating a CD16A and BCMA-mediated disease or a CD16A and B7H6-mediated disease.

56. The use according to claim 55, wherein the CD16A and BCMA-mediated disease or the CD16A and B7H6-mediated disease comprises a cancer.

57. The use according to claim 55, wherein the cancer comprises:
at least one of hemangioma, gastric cancer, liver cancer, lung cancer, breast cancer, colon cancer, nasopharyngeal cancer, bladder cancer, cervical cancer, prostate cancer, bone cancer, skin cancer, thyroid cancer, kidney cancer, esophageal cancer, melanoma, fibrosarcoma, rhabdomyosarcoma, astrocytoma, neuroblastoma, and glioma.

58. Use of the antibody or antigen-binding fragment according to any one of claims 1 to 18, the nucleic acid molecule according to claim 43, the expression vector according to claim 44, or the recombinant cell according to any one of claims 45 to 48 in the preparation of a kit for detecting CD16A.

59. Use of the bispecific binding molecule according to any one of claims 19 to 42, the nucleic acid molecule according to claim 43, the expression vector according to claim 44, or the recombinant cell according to any one of claims 45 to 48 in the preparation of a kit for detecting CD16A and/or BCMA, or CD16A and/or B7H6.

60. Use of the antibody or antigen-binding fragment according to any one of claims 1 to 18, the nucleic acid molecule according to claim 43, the expression vector according to claim 44, the recombinant cell according to any one of claims 45 to 48, the composition according to claim 49, or the medicament according to claim 50 in the prevention and/or treatment of a CD16A-mediated disease.

61. The use according to claim 60, wherein the CD16A-mediated disease comprises an autoimmune disease.

62. The use according to claim 61, wherein the autoimmune disease comprises:
at least one of systemic lupus erythematosus, rheumatoid arthritis, systemic vasculitis, scleroderma, dermatomyositis, autoimmune hemolytic anemia, thyroid autoimmune disease, ulcerative colitis, chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritis syndrome, pemphigus vulgaris, pemphigoid, primary biliary cirrhosis, multiple sclerosis, and acute idiopathic polyneuritis.

63. Use of the bispecific binding molecule according to any one of claims 19 to 42, the nucleic acid molecule according to claim 43, the expression vector according to claim 44, the recombinant cell according to any one of claims 45 to 48, the composition according to claim 49, or the medicament according to claim 50 in the prevention and/or treatment of a CD16A and BCMA-mediated disease or a CD16A and B7H6-mediated disease.

64. The use according to claim 63, wherein the CD16A and BCMA-mediated disease or the CD16A and B7H6-mediated disease comprises a cancer.

65. The use according to claim 64, wherein the cancer comprises:
at least one of hemangioma, gastric cancer, liver cancer, lung cancer, breast cancer, colon cancer, nasopharyngeal cancer, bladder cancer, cervical cancer, prostate cancer, bone cancer, skin cancer, thyroid cancer, kidney cancer, esophageal cancer, melanoma, fibrosarcoma, rhabdomyosarcoma, astrocytoma, neuroblastoma, and glioma.

66. A method for preventing and/or treating a CD16A-mediated disease, the method comprising:
administering to a patient a pharmaceutically acceptable dose of the antibody or antigen-binding fragment according to any one of claims 1 to 18, the nucleic acid molecule according to claim 43, the expression vector according to claim 44, the recombinant cell according to any one of claims 45 to 48, the composition according to claim 49, or the medicament according to claim 50.

67. A method for diagnosing a CD16A-mediated disease, the method comprising:
detecting CD16A in a sample to be tested using at least one of the antibody or antigen-binding fragment according to any one of claims 1 to 18, the nucleic acid molecule according to claim 43, the expression vector according to claim 44, or the recombinant cell according to any one of claims 45 to 48; and
determining, based on a detection result of the CD16A, a content of the CD16A in the sample to be tested.

68. The method according to claim 67, wherein the content of the CD16A in the sample to be tested being not lower than a minimum standard for the CD16A-mediated disease indicates that the sample to be tested is derived from a patient suffering from the CD16A-mediated disease.

69. A method for assessing a prognosis of a CD16A-mediated disease, the method comprising:
detecting CD16A in a sample to be tested using at least one of the antibody or antigen-binding fragment according to any one of claims 1 to 18, the nucleic acid molecule according to claim 43, the expression vector according to claim 44, or the recombinant cell according to any one of claims 45 to 48;
determining a content of the CD16A in the sample to be tested; and
assessing, based on the content of the CD16A, the prognosis of the CD16A-mediated disease.

70. The method according to claim 67 or 69, wherein the sample to be tested comprises at least one of blood, saliva, sweat, tissues, cells, serum, plasma, feces, and urine.

71. The method according to any one of claims 66, 67, or 69, wherein the CD16A-mediated disease comprises an autoimmune disease.

72. The method according to claim 71, wherein the autoimmune disease comprises:
at least one of systemic lupus erythematosus, rheumatoid arthritis, systemic vasculitis, scleroderma, dermatomyositis, autoimmune hemolytic anemia, thyroid autoimmune disease, ulcerative colitis, chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritis syndrome, pemphigus vulgaris, pemphigoid, primary biliary cirrhosis, multiple sclerosis, and acute idiopathic polyneuritis.

73. A method for preventing and/or treating a CD16A and BCMA-mediated disease or a CD16A and B7H6-mediated disease, the method comprising:
administering to a patient a pharmaceutically acceptable dose of the bispecific binding molecule according to any one of claims 19 to 42, the nucleic acid molecule according to claim 43, the expression vector according to claim 44, the recombinant cell according to any one of claims 45 to 48, the composition according to claim 49, or the medicament according to claim 50.

74. A method for diagnosing a CD16A and BCMA-mediated disease or a CD16A and B7H6-mediated disease, the method comprising:
detecting CD16A and BCMA, or CD16A and B7H6 in a sample to be tested using at least one of the bispecific binding molecule according to any one of claims 19 to 42, the nucleic acid molecule according to claim 43, the expression vector according to claim 44, or the recombinant cell according to any one of claims 45 to 48, and
determining, based on a detection result of CD16A and BCMA or a detection result of CD16A and B7H6, a content of CD16A and BCMA or a content of CD16A and B7H6 in the sample to be tested.

75. The method according to claim 74, wherein the content of CD 16A and BCMA or the content of CD16A and B7H6 in the sample to be tested being not lower than a minimum standard for CD16A and BCMA-mediated disease or CD16A and B7H6-mediated disease indicates that the sample to be tested is derived from a patient suffering from the CD16A and BCMA-mediated disease or the CD16A and B7H6-mediated disease.

76. A method for assessing a prognosis of a CD16A and BCMA-mediated disease or a CD16A and B7H6-mediated disease, the method comprising:
detecting CD16A and BCMA, or CD16A and B7H6 in a sample to be tested using at least one of the bispecific binding molecule according to any one of claims 19 to 42, the nucleic acid molecule according to claim 43, the expression vector according to claim 44, or the recombinant cell according to any one of claims 45 to 48;
determining a content of CD16A and BCMA or a content of CD16A and B7H6 in the sample to be tested, and
assessing, based on the content of CD16A and BCMA or the content of CD16A and B7H6, a prognosis of the CD16A and BCMA-mediated disease or the CD16A and B7H6-mediated disease.

77. The method according to claim 74 or 76, wherein the sample to be tested comprises at least one of blood, saliva, sweat, tissues, cells, serum, plasma, feces, and urine.

78. The method according to any one of claims 73, 74, or 76, wherein the CD16A and BCMA-mediated disease or the CD16A and B7H6-mediated disease comprises a cancer.

79. The method according to claim 78, wherein the cancer comprises:
at least one of hemangioma, gastric cancer, liver cancer, lung cancer, breast cancer, colon cancer, nasopharyngeal cancer, bladder cancer, cervical cancer, prostate cancer, bone cancer, skin cancer, thyroid cancer, kidney cancer, esophageal cancer, melanoma, fibrosarcoma, rhabdomyosarcoma, astrocytoma, neuroblastoma, and glioma.
